# EUROPEAN PATENT SPECIFICATION

(11) **EP 0 363 934 B1**
(45) Date of publication and mention of the grant of the patent: **29.12.1993**
(21) Application number: 89118906.0
(22) Date of filing: 11.10.1989
(51) Int. Cl.: C07C 69/732, C07C 69/675, C07C 59/11, C07D 209/24, C07D 209/12

(54) **Process for the preparation of 7-substituted-hept-6-enoic and -heptanoic acids and derivatives thereof**
Verfahren zur Herstellung von 7-substituierten Hept-6-en- und Heptansäuren und Derivaten davon
Procédé pour la préparation des acides hept-6-énoiques et heptanoiques substitués sur la position 7 et dérivés

(30) Priority: 13.10.1988 US 257475; 22.05.1989 US 355531
(43) Date of publication of application: 18.04.1990
(60) Divisional application: 93106005.7
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Chen, Kau-Ming, Randolph, N.J. 07869 (US); Kapa, Prasad Koteswara, Parsippany, N.J. 07054 (US); Lee, George T., Bloomfield, N.J. 07003 (US); Repic, Oljan, Hopatcong, N.J. 07843 (US); Hess, Petr, CH-4148 Pfeffingen (CH); Crevoisier, Michel, CH-4107 Ettingen (CH)

(56) References cited:
- EP-A- 0 244 364
- GB-A- 945 536
- US-A- 4 650 890
- US-A- 4 739 073
- TETRAHEDRON LETTERS, vol. 28, no. 2, 1987, pages 155-158, Oxford, GB, K.-M. CHENG et al.: "1.3-Syn diastereoselective reduction of beta-hydroxyketones utilizing alkoxydialkylboranes"
- CHEMISTRY LETTERS, 1987, pages 1923-1926, The Chemical Society of Japan; K.M. CHEN et al.: "A Novel Method for the In Situ Generation of Alkoxydialkylboranes and Their Use in the Selective Preparation of 1,3-Syn Diols"

## Description

The invention relates to the preparation of 7-substituted-hept-6-enoic and -heptanoic acids and derivatives and intermediates thereof.

### 1. Object

The invention concerns a process for the preparation of a compound of formula I 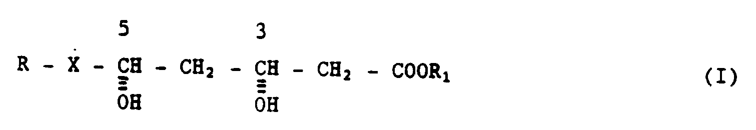
wherein
- X: is -CH₂CH₂-or -CH=CH-;
- R₁: is an ester group inert to the reaction conditions; and
- R: is an organic radical having groups which are inert under reducing conditions.
with the proviso that, when R is triphenylmethyl, then R₁ additionally includes allyl and X is -OCH₂-.

The common feature linking these various process steps is that they all lead to improvements at various stages in the preparation of 7-substituted-hept-6-enoic and -heptanoic acid end products and derivatives thereof which are cholesterol biosynthesis inhibitors. This link is illustrated hereunder with a specific embodiment concerning the preparation of one specific cholesterol biosynthesis inhibitor, namely erythro-(E)-3,5-dihydroxy-7-[3'-(4''-fluorophenyl)-1'-(1''-methylethyl)indol-2'-yl]hept-6-enoic acid in racemic or optically pure form; in free acid, salt, ester or δ-lactone, i.e. internal ester, form.

The process of the invention for the preparation of a compound of formula I comprises stereoselectively reducing a racemic or optically pure compound of formula II 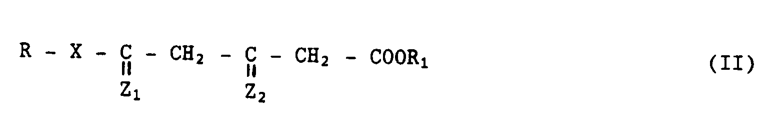
wherein
R, R₁ and X are as defined above and
one of Z₁ and Z₂ is oxygen and the other is hydroxy and hydrogen, to produce a corresponding compound of formula I.

As appears from formula I the compounds have the syn, i.e. the erythro configuration.

The symbol (E)- appearing at the beginning of a formula or in a name indicates that a double bond is in the trans configuration.

The invention allows the preparation of a compound of formula I as defined above in a state of optical purity such that the proportion of erythro to threo isomer is 99.1:0.9 or higher, preferably 99.5:0.5 or higher; especially 99.7 : 0.3 or higher.

The compounds of formula I, which are esters, and the corresponding free acids, salts and cyclic esters (δ-lactones) are pharmaceuticals, in particular HMG-CoA reductase inhibitors, i.e. cholesterol biosynthesis inhibitors and, therefore, they are indicated for use for the treatment of hypercholesterolemia, hyperlipoproteinemia and atherosclerosis.

### 2. Preferred significances

An ester group R₁ preferably is a physiologically acceptable and hydrolyzable ester group when the desired end product is an ester.

By the term "physiologically acceptable and hydrolyzable ester group" is meant a group which, together with the -COO- radical to which it is attached, forms an ester group which is physiologically acceptable and hydrolyzable under physiological conditions to yield the corresponding carboxylic acid of the compound of formula I (i.e. wherein R₁ is replaced by hydrogen) and an alcohol which itself is physiologically acceptable, i.e. non-toxic at the desired dosage level, particularly a group which is free of centers of asymmetry.

R₁ is preferably R₂ where R₂ is C₁₋₄alkyl or benzyl, especially R₂' where R₂' is C₁₋₃alkyl, n-butyl, i-butyl, t-butyl or benzyl, e.g. ethyl, preferably isopropyl or t-butyl, especially t-butyl. It may also be allyl when R is triphenylmethyl and X is - OCH₂-.

X preferably is X' where X' is - CH=CH-, preferably (E)-CH=CH-.

R preferably is selected from a group A, B, C, D, Ea, Eb, Ec, F, G, H, J, K, L, M or N as follows:
- A: phenyl trisubstituted by R₁ₐ, R₂ₐ and R₃ₐ wherein R₁ₐ, R₂ₐ and R₃ₐ are independently hydrogen; halo; C₁₋₄alkyl; C₁₋₄haloalkyl; phenyl; phenyl substituted by halo, C₁₋₄alkoxy, C₂₋₈alkanoyloxy, C₁₋₄alkyl or C₁₋₄haloalkyl; or -OR₄ₐ wherein R₄ₐ is hydrogen, C₂₋₈alkanoyl, benzoyl, phenyl, halophenyl, phenyl(C₁₋₃alkyl), C₁₋₉alkyl, cinnamyl, C₁₋₄haloalkyl, allyl, cycloalkyl(C₁₋₃alkyl), adamantyl(C₁₋₃alkyl) or substituted phenyl(C₁₋₃alkyl) each substituent of which is selected from halo, C₁₋₄alkoxy, C₁₋₄alkyl and C₁₋₄haloalkyl; whereby the halogen atoms are fluoro or chloro and cycloalkyl includes cyclohexyl;
- B: 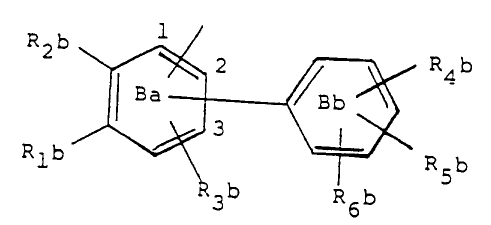 wherein R₁b and R₂b together form & radical of formula:
a) 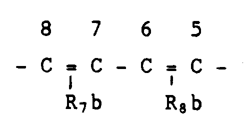 or b) - CH₂-CH₂-CH₂-CH₂-
wherein
- R₃b: is hydrogen, C₁₋₃alkyl, C₁₋₃alkoxy, trifluoromethyl, fluoro, chloro, phenoxy or benzyloxy;
- R₄b: is hydrogen, C₁₋₃alkyl, n-butyl, i-butyl, C₁₋₃alkoxy, n-butoxy, i-butoxy, trifluoromethyl, fluoro, chloro, phenoxy or benzyloxy;
- R₅b: is hydrogen, C₁₋₃alkyl, C₁₋₃alkoxy, trifluoromethyl, fluoro, chloro, phenoxy or benzyloxy;
with the provisos that not more than one of R₄b and R₅b is trifluoromethyl, not more than one of R₄b and R₅b is phenoxy, and not more than one of R₄b and R₅b is benzyloxy;
- R₆b: is hydrogen, C₁₋₂alkyl, C₁₋₂alkoxy, fluoro or chloro;
- R₇b: is hydrogen, C₁₋₃alkyl, n-butyl, i-butyl, C₁₋₃alkoxy, n-butoxy, trifluoromethyl, fluoro, chloro, phenoxy or benzyloxy;
- R₈b: is hydrogen, C₁₋₃alkyl, C₁₋₃alkoxy, trifluoromethyl, fluoro, chloro, phenoxy or benzyloxy;
with the provisos that not more than one of R₇b and R₈b is trifluoromethyl, not more than one of R₇b and R₈b is phenoxy, and not more than one of R₇b and R₈b is benzyloxy;
with the further proviso that the free valences on rings Ba and Bb are ortho to each other;
- C: 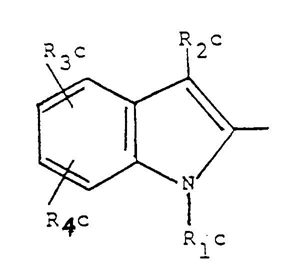 wherein
- one of R₁c and R₂c: is phenyl substituted by R₅c, R₆c and R₇c and the other is C₁₋₃alkyl, n-butyl or i-butyl,
- R₃c: is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy,
- R₄c: is hydrogen, C₁₋₃alkyl, C₁₋₃alkoxy, trifluoromethyl, fluoro, chloro, phenoxy or benzyloxy,
with the provisos that not more than one of R₃c and R₄c is trifluoromethyl, not more than one of R₃c and R₄c is phenoxy and not more than one of R₃c and R₄c is benzyloxy,
- R₅c: is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy,
- R₆c: is hydrogen, C₁₋₃alkyl, C₁₋₃alkoxy, trifluoromethyl, fluoro, chloro, phenoxy or benzyloxy,
with the provisos that not more than one of R₅c and R₆c is trifluoromethyl, not more than one of R₅c and R₆c is phenoxy and not more than one of R₅c and R₆c is benzyloxy, and
- R₇c: is hydrogen, C₁₋₂alkyl, C₁₋₂alkoxy, fluoro or chloro;
- D: 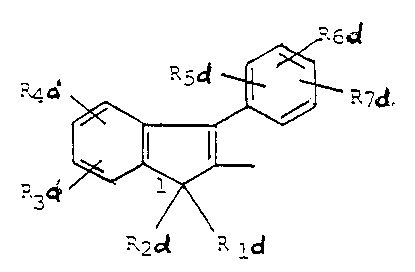 wherein
- R₁d: is hydrogen or primary or secondary C₁₋₆alkyl not containing an asymmetric carbon atom and
- R₂d: is primary or secondary C₁₋₆alkyl not containing an asymmetric carbon atom or
- R₁d and R₂d: taken together are -(CH₂)ₘ-or(Z)-CH₂-CH=CH-CH₂-wherein m is 2, 3, 4, 5 or 6;
- R₃d: is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy,
- R₄d: is hydrogen, C₁₋₃alkyl, C₁₋₃alkoxy, trifluoromethyl, fluoro, chloro, phenoxy or benzyloxy,
with the provisos that not more than one of R₂d and R₃d is trifluoromethyl, not more than one of R₂d and R₃d is phenoxy and not more than one of R₂d and R₃d is benzyloxy,
- R₅d: is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy,
- R₆d: is hydrogen, C₁₋₃alkyl, C₁₋₃alkoxy, trifluoromethyl, fluoro, chloro, phenoxy or benzyloxy,
with the provisos that not more than one of R₅d and R₆d is trifluoromethyl, not more than one of R₅d and R₆d is phenoxy and not more than one of R₅d and R₆d is benzyloxy,
- R₇d: is hydrogen, C₁₋₂alkyl, C₁₋₂alkoxy, fluoro or chloro;
- Ea: 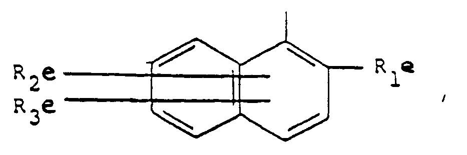
- Eb: 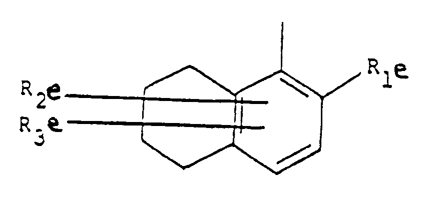 or
- Ec: 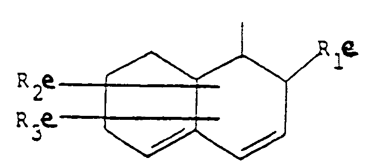 wherein
- each of R₁e, R₂e and R₃e: is independently fluoro, chloro, hydrogen or C₁₋₄alkyl, R₁e preferably being methyl;
- F: 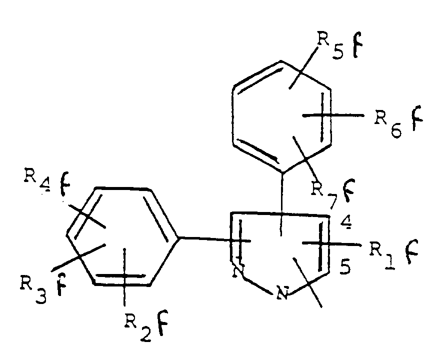 wherein
- R₁f: is C₁₋₆alkyl not containing an asymmetric carbon atom,
- each of R₂f and R₅f: is independently hydrogen, C₁₋₃alkyl, n-butyl, i-butyl, t-butyl, C₁₋₃alkoxy, n-butoxy, i-butoxy, trifluoromethyl, fluoro, chloro, phenyl, phenoxy or benzyloxy,
- each of R₃f and R₆f: is independently hydrogen, C₁₋₃alkyl, C₁₋₃alkoxy, trifluoromethyl, fluoro, chloro, phenoxy or benzyloxy and
- each of R₄f and R₇f: is independently hydrogen, C₁₋₂alkyl, C₁₋₂alkoxy, fluoro or chloro,
with the provisos that not more than one of R₂f and R₃f is trifluoromethyl, not more than one of R₂f and R₃f is phenoxy, not more than one of R₂f and R₃f is benzyloxy, not more than one of R₅f and R₆f is trifluoromethyl, not more than one of R₅f and R₆f is phenoxy and not more than one of R₅f and R₆f is benzyloxy;
with the provisos that
(i) the free valence of the pyrazole ring is in the 4- or 5-position, and
(ii) the R₁f group and the free valence are ortho to each other;
- G: 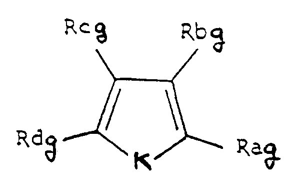 wherein
- Rag: is a single bond to X, Rbg is R₂g, Rcg is R₃g, Rdg is R₄g and K is 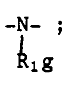 or
- Rag: is R₁g, Rbg is a single bond to X, Rcg is R₂g, Rdg is R₃g, and K is O, S or 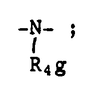
- R₁g, R₂g, R₃g and R₄g: independently are C₁₋₆alkyl not containing an asymmetric carbon atom, C₃₋₇cycloalkyl or phenyl substituted by R₅g, R₆g and R₇g, or in the case of R₃g and R₄g additionally hydrogen, or for R₃g when K is O or S and X is X' additionally Ga, and
- Ga: is -C(R₁₇g)=C(R₁₈g)R₁₉g wherein
- R₁₇g: is hydrogen or C₁₋₃alkyl and
- R₁₈g and R₁₉g: are independently hydrogen, C₁₋₃alkyl or phenyl,
- each R₅g: is independently hydrogen, C₁₋₃alkyl, n-butyl, i-butyl, t-butyl, C₁₋₃alkoxy, n-butoxy, i-butoxy, trifluoromethyl, fluoro, chloro, bromo, phenyl, phenoxy or benzyloxy;
- each R₆g: is independently hydrogen, C₁₋₃alkyl, C₁₋₃alkoxy, trifluoromethyl, fluoro, chloro, bromo, phenoxy or benzyloxy; and
- each R₇g: is independently hydrogen, C₁₋₂alkyl, C₁₋₂alkoxy, fluoro or chloro,
with the proviso that there may only be one each of trifluoromethyl, phenoxy and benzyloxy on each phenyl ring substituted by R₅g, R₆g and R₇g;
- H: 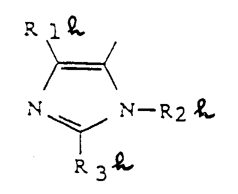 wherein
- R₁h: is C₁₋₆alkyl not containing an asymmetric carbon atom, C₃₋₇cycloalkyl, adamantyl-l or phenyl substituted by R₄h, R₅h and R₆h;
- R₂h: is C₁₋₆alkyl not containing an asymmetric carbon atom, C₃₋₇cycloalkyl, adamantyl-l or phenyl substituted by R₇h, R₈h and R₉h;
- R₃h: is hydrogen, C₁₋₆alkyl not containing an asymmetric carbon atom, C₃₋₇cycloalkyl, adamantyl-l, styryl or phenyl substituted by R₁₀h, R₁₁h and R₁₂h;
- each of R₄h, R₇h and R₁₀h: is independently hydrogen, C₁₋₃alkyl, n-butyl, i-butyl, t-butyl, C₁₋₃alkoxy, n-butoxy, i-butoxy, trifluoromethyl, fluoro, chloro, bromo, phenyl, phenoxy or benzyloxy,
- each of R₅h, R₆h and R₁₁h: is independently hydrogen, C₁₋₃alkyl, C₁₋₃alkoxy, trifluoromethyl, fluoro, chloro, bromo, -COOR₁₇h, -N(R₁₉h)₂, phenoxy or benzyloxy, wherein R₁₇h is hydrogen, R₁₈h or M, wherein
- R₁₈h: is C₁₋₃alkyl, n-butyl, i-butyl, t-butyl or benzyl, and
- M: is as defined above, and
- each R₁₉h: is independently C₁₋₆alkyl not containing an asymmetric carbon atom, and
- each of R₆h, R₉h and R₁₂h: is independently hydrogen, C₁₋₂alkyl, C₁₋₂alkoxy, fluoro or chloro,
with the provisos that not more than one substituent on each phenyl ring independently is trifluoromethyl, not more than one substituent on each phenyl ring independently is phenoxy, and not more than one substituent on each phenyl ring independently is benzyloxy;
- J: 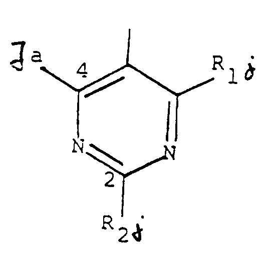 wherein
- each of R₁j and R₂j: is independently C₁₋₆alkyl not containing an asymmetric carbon atom, C₃₋₆cycloalkyl or phenyl-(CH₂)ₘ-wherein m is 0, 1, 2 or 3 and the phenyl group is unsubstituted or substituted by any of R₃j, R₄j and R₅j wherein R₃j, R₄j and R₅j are as defined below; or
- R₂j: is -Yj-benzyl, - N(R₈j)₂ or Ja wherein
- Yj: is -O-or -S-;
- each R₈j: is independently C₁₋₄alkyl not containing an asymmetric carbon atom or may form part of a 5-, 6-, or 7-membered ring Jb, ring Jb being substituted or unsubstituted and optionally also containing one or more heteroatoms; and
- Ja: is Ja' or Ja'' wherein
- Ja': is a heterocyclic group which is unsubstituted or substituted by one or two C₁₋₂alkyl or C₁₋₂alkoxy groups and
- Ja'': is Ja''a or Ja''b 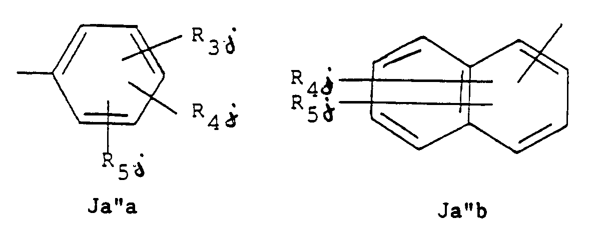 wherein
- R₃j: is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy,
- R₄j: is hydrogen, C₁₋₃alkyl, C₁₋₃alkoxy, trifluoromethyl, fluoro, chloro, phenoxy or benzyloxy and
- R₅j: is hydrogen, C₁₋₂alkyl, C₁₋₃alkoxy, fluoro or chloro;
with the provisos that not more than one of R₃J and R₄j is trifluoromethyl, not more than one of R₃j and R₄l is phenoxy and not more than one of R₃j and R₄j is benzyloxy;
- K: 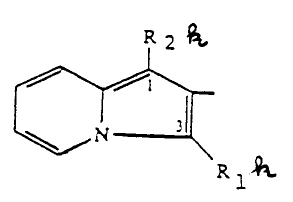 wherein
- each of R₁k and R₂k: is independently
(a) phenyl substituted by R₅k, R₆k and R₇k wherein
   R₅k is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy;
   R₆k is hydrogen, C₁₋₃alkyl, C₁₋₃alkoxy, fluoro, or chloro; and
   R₇k is hydrogen, C₁₋₂alkyl, C₁₋₂alkoxy, fluoro or chloro;
(b) hydrogen or a primary or secondary C₁₋₆alkyl not containing an asymmetric carbon atom;
(c) C₃₋₆cycloalkyl; or
(d) phenyl(CH₂)ₘ-wherein m is 1, 2 or 3;
- L: 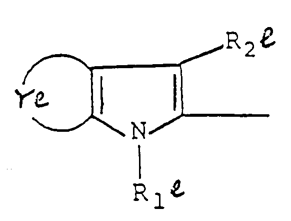 wherein
- Yl: is

- CH=CH- CH=N- , -CH=CH- N=CH- , -CH=N- CH=CH- or -N=CH- CH=CH- ,
- R₁l: is primary C₁₋₆alkyl not containing an asymmetric carbon atom; or isopropyl;
- R₂l: is:
a) phenyl substituted by R₅l, R₆l and R₇l wherein R₅l is t-butyl, C₁₋₃alkoxy, n-butoxy, i-butoxy, trifluoromethyl, fluoro, chloro, phenoxy or benzyloxy;
   R₆l is hydrogen, C₁₋₃alkyl, C₁₋₃alkoxy, trifluoromethyl, fluoro, chloro, phenoxy or benzyloxy;
   with the provisos that not more than one of R₃l and R₆l is trifluoromethyl, not more than one of R₅l and R₆l is phenoxy, and not more than one of R₅l and R₆l is benzyloxy,
   R₇l is hydrogen, C₁₋₂alkyl, C₁₋₂alkoxy, fluoro or chloro,
b) a primary or secondary C₁₋₆alkyl not containing an asymmetric carbon atom,
c) C₃₋₆cycloalkyl or
d) phenyl-(CH₂)ₘ-wherein m is 1, 2 or 3;
- M: 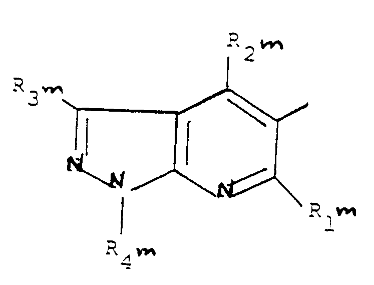 wherein
- R₁m: is C₁₋₆alkyl not containing an asymmetric carbon atom, C₅₋₇cycloalkyl, (C₅₋₇cycloalkyl)methyl, phenyl-(CH₂)ₘ-, pyridyl-2, pyridyl-3, pyridyl-4, thienyl-2, thienyl-3 or phenyl substituted by R₅m, R₆m and R₇m;
- R₂m: is C₁₋₆alkyl not containing an asymmetric carbon atom, C₅₋₇cycloalkyl, (C₅₋₇cycloalkyl)methyl, phenyl-(CH₂)ₘ-, pyridyl-2, pyridyl-3, pyridyl-4, thienyl-2, thienyl-3 or phenyl substituted by R₈m, R₉m and R₁₀m,
with the proviso that not more than one of R₁m and R₂m is a member of the group consisting of pyridyl-2, pyridyl-3, pyridyl-4, thienyl-2, thienyl-3, phenyl substituted by R₅m, R₆m and R₇m and phenyl substituted by R₆m, R₉m and R₁₀m;
- R₃m: is C₁₋₆alkyl not containing an asymmetric carbon atom, C₅₋₇cycloalkyl or phenyl substituted by R₁₁m, R₁₂m and R₁₃m;
- R₄m: is C₁₋₆alkyl not containing an asymmetric carbon atom, C₅₋₇cycloalkyl or phenyl substituted by R₁₄m, R₁₅m and R₁₆m; wherein
- each of R₅m, R₈m, R₁₁m and R₁₄m: is independently hydrogen, C₁₋₃alkyl, n-butyl, i-butyl, t-butyl, C₁₋₃alkoxy, n-butoxy, i-butoxy, trifluoromethyl, fluoro, chloro, bromo, phenyl, phenoxy or benzyloxy,
- each of R₆m, R₉q, R₁₂m and R₁₅m: is independently hydrogen, C₁₋₃alkyl, C₁₋₃alkoxy, trifluoromethyl, fluoro, chloro, phenoxy or benzyloxy, and
- each of R₇m, R₁₀m, R₁₃m and R₁₆m: is independently hydrogen, C₁₋₂alkyl, C₁₋₂alkoxy, fluoro or chloro,
with the provisos that not more than one substituent on each phenyl ring independently is trifluoromethyl, not more than one substituent on each phenyl ring independently is phenoxy, and not more than one substituent on each phenyl ring independently is benzyloxy;
- N: 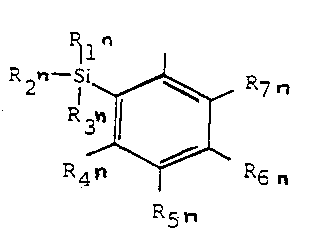 wherein
- each of R₁n, R₂n and R₃n: is independently alkyl of l to 4 carbon atoms; or phenyl which may be unsubstituted or substituted either by one or two alkyl or alkoxy groups of l to 3 carbon atoms, or chloro; or by one fluoro, bromo or trifluoromethyl substituent;
- R₄n: is hydrogen or alkyl of l to 3 carbon atoms, e.g. methyl;
- R₅n: is hydrogen, lower alkyl or alkoxy; halo, trifluoromethyl; or phenyl, benzyl, or benzyloxy, wherein the aromatic portion may be unsubstituted or substituted by up to two groups, one of which may be fluoro, bromo or trifluoromethyl; or one or two of which may be lower alkyl or alkoxy, or chloro;
- R₆n: is hydrogen, lower alkyl or alkoxy, halo, or trifluoromethyl; and
- R₇n: is hydrogen, lower alkyl or alkoxy, halo or trifluoromethyl; and
- any of R₄n + R₅n, R₅n + R₆n, or R₆n + R₇n: may constitute either a - CH=CH-CH=CH-or a - (CH₂)₄-radical to form a ring which is substituted by R₈n which is hydrogen; halo, or lower alkyl or alkoxy;
provided that there be no more than one trifluoromethyl group and no more than two bromo substituents present on the molecule.

The compounds of formula I may be divided into thirteen groups, i.e., groups IA to IN, depending upon the significance of R, i.e.:
IA when R = A,
IB when R = B,
IC when R = C,
ID when R = D,
IE when R = Ea, Eb or Ec,
IF when R = F,
IG when R = G,
IH when R = H,
IJ when R = J,
IK when R = K,
IL when R = L,
IM when R = M and
IN when R = N.

### 3. Stereochemistry

Generally, when a hydroxy-keto compound of formula II is reduced to a dihydroxy compound of formula I, an additional center of asymmetry is generated. Consequently, when a racemic compound of formula II is utilized, four stereoisomers (comprising two pairs of enantiomers, i.e. a pair of erythro and a pair of threo enantiomers) of the resulting compound of formula I are formed. Alternatively, when an optically pure compound of formula II is utilized, two diastereoisomers (i.e. one erythro and one threo isomer) of the compound of formula I are formed, e.g., the 3R,5S and 3S,5S diastereoisomers which result from the reduction of the 5S hydroxy compound. Diastereoisomers may be separated by conventional means, such as by fractional crystallization, column chromatography, preparative thin layer chromatography or HPLC. The proportion of erythro to threo isomer obtained by these methods is usually variable and can be e.g. up to about 98 : 1.

With the stereoselective process of the present invention, when a racemic compound of formula II is utilized, only two stereoisomers (comprising the erythro pair of enantiomers) of the resulting compound of formula I are formed almost exclusively. Alternatively, when an optically pure compound of formula II is utilized, only one enantiomer of the compound of formula I is formed almost exclusively, and this enantiomer is the corresponding erythro enantiomer. For example, the 3R,5S enantiomer results from the reduction of the 5S hydroxy compound wherein X is -CH=CH-.

The proportion of erythro to threo isomer obtained with the process of the present invention is about 99.1 : 0.9 or higher, particularly about 99.5 : 0.5 or higher, especially about 99.7 : 0.3 or higher.

The term "stereoselective" as used herein thus means that the proportion of the erythro to the threo form is 99.1:0.9 or higher.

The stereoisomers of the compounds of formula I wherein X is -CH=CH-according to the present invention are the 3R,5S and the 3S, 5R isomer and the racemate consisting of both of them, of which the 3R,5S isomer and the racemate are preferred.

The stereoisomers of the compounds of formula I wherein X is -CH₂CH₂- according to the present invention are the 3R,5R and the 3S,5S isomer and the racemate consisting of both of them, of which the 3R,5R isomer and the racemate are preferred.

### 4. State of the art

Conventional processes for reducing the keto group of a compound of formula II have employed mild reducing agents such as sodium borohydride or a complex of t-butylamine and borane, in an inert organic solvent such as a lower alkanol, to yield a mixture of diastereomeric forms from the optically pure starting compound, or alternatively, the racemic diastereoisomers from the racemic starting material.

A three-step, partly stereoselective reduction process has been used to obtain predominantly the erythro racemate from the racemic starting material. In the **first step**, a compound of formula II is contacted either with a trialkylborane compound or a compound of formula III:

R₄O-B-(R₃)₂ (III)

in which R₄ is allyl or lower alkyl having from l to 4 carbon atoms, preferably not tertiary, and R₃ is a primary or secondary alkyl having 2 to 4 carbon atoms, preferably not tertiary, in a reaction medium comprising an alcohol and tetrahydrofuran (THF). In the **second step** of such processes, sodium borohydride (NaBH₄) is added to the reaction medium, and reaction proceeds with the reduction of the keto group, and in turn to the formation of the cyclic boronate or a borane complex of the compound of formula I. In the **third step**, the reaction mixture containing the boron complex and/or cyclic boronate ester is azeotroped with methanol or ethanol, or alternatively, is treated in an organic solvent with aqueous peroxy compound, such as a peroxide, e.g. hydrogen peroxide, or a perborate, e.g. sodium perborate, to yield the resulting compounds of formula I. The aforementioned process is said to provide the erythro racemate with, e.g., about 98 % selectivity relative to the threo isomers (Chen et al., Tetrahedron Letters 28, 155 [1987]).

### 5. Detailed description

The process of the present invention comprises a method for stereoselectively reducing racemic and optically pure compounds of formula II to obtain almost exclusively the erythro isomers of formula I. Advantageously, the reduction of the keto group of the compound of formula II occurs virtually instantaneously. The compounds of formula I, i.e. the erythro isomers, are, additionally, provided in increased chemical purity and may be further enriched to above 99 % chemical purity by simple recrystallization.

According to a **first step** of the process of the present invention **[step (a)],** a compound of formula III is mixed with sodium borohydride NABH₄ in a reaction medium comprising an alcohol and tetrahydrofuran.

In a **second step [step (b)],** a compound of formula II is treated with the mixture obtained in step (a) under conditions suitable to obtain a mixture containing a cyclic boronate compound of formula IV(a) 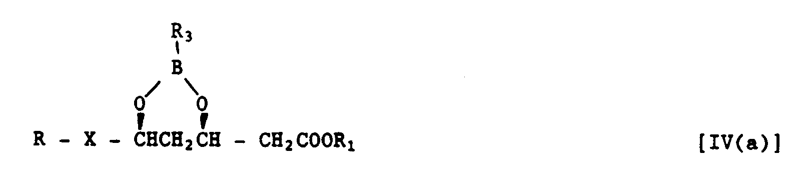
and/or a boron complex of formula IV(b) 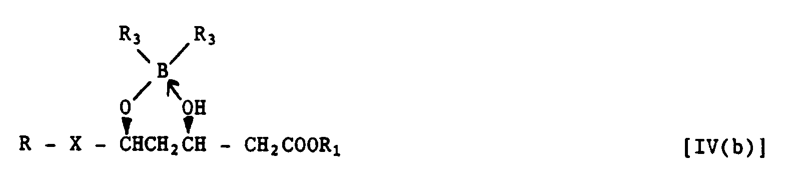
wherein R, R₁, R₃ and X are as defined above. The latter predominates prior to quenching. However, quenching converts the boron complex into boronate ester.

In a **third step [step (c)],** the product obtained in step (b) is cleaved to obtain a corresponding compound of formula I.

**Step (a)** is preferably carried out under essentially anhydrous conditions, preferably in an inert atmosphere, at from about -100° to about +30°C, preferably at from about -80° to about -60 °C, especially at from about -78° to about -70°C. The reaction medium employed in step (a) comprises a mixture of alcohol and tetrahydrofuran wherein the alcohol is of formula AlkOH, in which Alk is alkyl of l to 4 carbon atoms, e.g. methyl or ethyl, preferably not tertiary.

One of the products of step (a) may be R₄OH, derived from the compound of formula III employed. However, it is not necessary that all or part of Alk be the same as R₄. The sodium borohydride should generally be present in at least equimolar amount with the compound of formula II, and more preferably in slight excess, such as e.g. from about 1.1:1 to about 1.5 : 1 moles NaBH₄ per mole of ketone. The molar ratio of the compound of formula III to the compound of formula II is at least about 0.5 : 1, and more preferably from about 0.7 : 1 to about 1.5 : 1 moles of borane compound per mole of ketone.

**Step (b)** is also preferably carried out at reduced temperatures, the internal temperature being maintained at about -100° to about -40°C, especially from about -78° to about -70°C. The compound of formula II is preferably in a solvent such as alcohol/THF or THF. Preferably the reaction medium of step (a) and the solvent of the compound of formula II which is added in step (b) are selected to make up a combined medium wherein the ratio (v/v) of alcohol to tetrahydrofuran is from about l:3 to about l:6 of alcohol to THF, especially from about l:3 to about 1:4. Reduction of the keto group is exothermic and occurs rapidly, and therefore, addition of the keto compound is desirably staged in order to maintain an internal temperature in the range of from about -78° to about -70°C. The reduction is almost instantaneous and the reaction mixture is then quenched by adding, e.g., aqueous sodium bicarbonate, ammonium chloride or acetic acid, and a mixture of the desired cyclic boronate intermediate is obtained.

In **step (c)** the reaction product of step (b) may be azeotroped with methanol or ethanol, at, e.g., from about 60° to about 80°C, under essentially anhydrous conditions. Alternatively and preferably, particularly where X is -CH=CH-, the product having been neutralized by addition of sodium bicarbonate (NaHCO₃) is dissolved in an organic solvent, e.g., ethyl acetate, and treated with aqueous (e.g. 30 %) hydrogen peroxide or aqueous sodium perborate (NaBO₃.4H₂O), initially at reduced temperature, e.g. about +10°C, and then allowed to warm to a moderate temperature, e.g. about 20° to about 30°C, to obtain the corresponding compound of formula I.

Alternatively, the cyclic boronate ester from step (b) may be extracted with an organic solvent such as ethyl acetate and then treated directly with an aqueous solution of a peroxy compound such as 30 % aqueous hydrogen peroxide or aqueous sodium perborate solution to obtain the corresponding compound of formula I.

Since reducing conditions occur in practicing the invention, it is understood that any substituents or functions on the radical employed as R will be inert, i.e. that it will be free of substituents or functions which would be reactive or susceptible of alteration under such conditions, e.g. by known methods of masking or protecting such functions or introducing them at a later stage.

The compounds of formula I, the corresponding δ-lactones, free acids and salts, processes for converting a compound of formula I wherein R₁ has one significance into the corresponding compound wherein it has a different significance and/or into the corresponding δ-lactone or free acid or salts are known.

The compounds of formula I may if desired be converted by conventional means into corresponding free acid or salt forms, i.e. wherein R₁ is replaced by hydrogen or a cation, such as an alkali metal cation or ammonium, preferably sodium or potassium and especially sodium, into other ester forms, or into the corresponding δ-lactones, i.e. internal esters.

As mentioned above the compounds of formula I obtained according to the process of the invention are pharmaceuticals. In a further embodiment, however, the process of the invention may also be applied to the preparation of chiral intermediates, of e.g. formula Iu 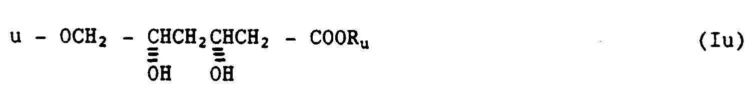
wherein
- u: is triphenylmethyl (trityl) and
- Rᵤ: is allyl or a radical forming an ester inert under the reaction conditions, preferably allyl or C₁₋₃alkyl, n-butyl, i-butyl, t-butyl or benzyl, especially t-butyl,
by stereoselectively reducing a racemic or optically pure compound of formula IIu 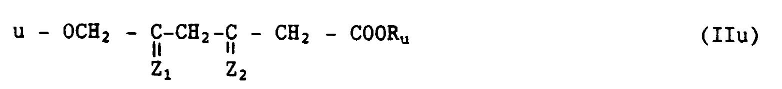
wherein u, Rᵤ, Z₁ and Z₂ are as defined above.

Such chiral intermediates are disclosed in e.g. EP 244364. They are indicated for use in preparing pharmaceuticals.

### 6. Starting materials

The (primary or secondary C₁₋₄alkoxy or allyloxy)-di-(primary C₂₋₄alkyl)boranes of formula III used as starting materials in the process of the present invention are known [Koster et al, Ann. (l975), 352; Chen et al., Tetrahedron Letters 28, l55 (l987); and Chen et al, Chemistry Letters (1987), l923-l926]. However, they may be prepared in situ from the corresponding tri-(primary or secondary C₂₋₄alkyl)boranes by reaction with a primary or secondary C₁₋₄alkanol or allyl alcohol, the concentration of the former in the latter preferably being from about 0.2 M to about l.2 M, especially about 0.5 M.

The compounds of formula II are known or may be prepared analogously to known compounds of formula II, e.g. as described in USP 4 739 073 (e.g. for Z₂ = oxygen) or in EP 216 785 (e.g. for Z₁ = oxygen).

Thus the compounds of formula II wherein Z₂ is oxygen are normally prepared by reaction of a compound of formula V

R - X - CHO (V)

wherein R and X are as defined above,
with the dianion of a compound of formula VI

CH₃ - CO - CH₂ - COOR₁ (VI)

wherein R₁ is as defined above.

### 7. Further embodiments concerning earlier intermediates

The compounds of formula V and VI are also known. In further embodiments, however, the present invention also comprises the preparation of the compounds of the invention via a novel process for the preparation of a subgroup of compounds of formula V, namely the compounds of formula Va 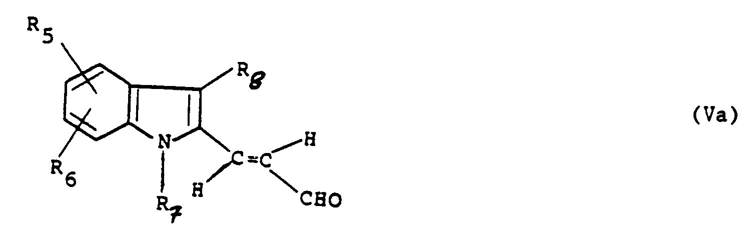
wherein
- R₅: is hydrogen, C₁₋₃alkyl, n-butyl, i-butyl, t-butyl, C₃₋₆cycloalkyl, C₁₋₃alkoxy, n-butoxy, i-butoxy, trifluoromethyl, fluoro, chloro, phenoxy or benzyloxy;
- R₆: is hydrogen, C₁₋₃alkyl, C₁₋₃alkoxy, trifluoromethyl, fluoro, chloro, phenoxy or benzyloxy;
with the provisos that not more than one of R₅ and R₆ is trifluoromethyl, not more than one of R₅ and R₆ is phenoxy, and not more than one of R₅ and R₆ is benzyloxy;
- one of R₇ and R₈: is phenyl trisubstituted by R₉, R₁₀ and R₁₁ and the other is primary or secondary C₁₋₆alkyl not containing an asymmetric carbon atom, C₃₋₆cycloalkyl or phenyl-(CH₂)ₘ-,
wherein
- R₉: is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy;
- R₁₀: is hydrogen, C₁₋₃alkyl, C₁₋₃alkoxy, trifluoromethyl, fluoro, chloro, phenoxy or benzyloxy;
- R₁₁: is hydrogen, C₁₋₂alkyl, C₁₋₂alkoxy, fluoro or chloro, and
- m: is 1, 2 or 3;
with the provisos that not more than one of R₉ and R₁₀ is trifluoromethyl, not more than one of R₉ and R₁₀ is phenoxy, and not more than one of R₉ and R₁₀ is benzyloxy; starting from a subgroup of compounds of formula VII

(E) - OHC - CH=CH - N(R₁₂)R₁₃ (VII)

wherein
- R₁₂: is C₁₋₃alkyl, phenyl or phenyl substituted by 1 to 3 substituents each of which is independently C₁₋₃alkyl, C₁₋₃alkoxy, fluoro, chloro, bromo or nitro with a maximum of two nitro groups; and
- R₁₃: independently has the significance indicated above for R₁₂,
as well as via a novel process for the preparation of the compounds of formula VII themselves.

The process for the preparation of the compounds of formula VII is hereinafter designated as "**process A**" and the process for the preparation of the compounds of formula Va is hereinafter designated as "**process B**".

The compounds of formulae Va, VII and XVII (see under 7.2.) are known from i.a. USP 4 739 073 which discloses the compounds of formula VII wherein R₁₂ is C₁₋₃alkyl and their use for the synthesis of the compounds of formula Va, the compounds of formula XVII, and the use of the compounds of formula Va for the synthesis of indole analogs of mevalonolactone and derivatives thereof which are indicated for use as HMG-CoA reductase inhibitors. Since they inhibit cholesterol synthesis, they lower the blood cholesterol level and are therefore indicated for use in the treatment of hypercholesterolemia, hyperlipoproteinemia and atherosclerosis.

Compounds of formula VII and their synthesis are also disclosed in British Patent Specification No. 945 536 and Czechoslovakian Patent No. 90 045. However, the processes disclosed therein differ from process A with respect to, for example, the use of phosgene or phosphorus trichloride, pentachloride or oxychloride rather than an oxalic acid derivative.

### 7.1. Process A (preparation of the compounds of formula VII)

The process for the preparation of the compounds of formula VII **(process A)** comprises
(i) reacting a compound of formula VIII

   OHC - N(R₁₂)R₁₃ (VIII)

   wherein R₁₂ and R₁₃ are as defined above,
   with a compound of formula IX

   Xₐ - CO-CO - Xₐ (IX)

   wherein Xₐ is a monovalent leaving group,
   optionally in an inert anhydrous organic medium, to form the corresponding compound of formula X

   Xₐ - CH=N⁺(R₁₂)R₁₃ Xₐ- (X)

   wherein Xₐ, R₁₂ and R₁₃ are as defined above,
(ii) reacting that compound of formula X
   with a compound of formula XI

   R₁₄O - CH=CH₂ (XI)

   wherein R₁₄ is a monovalent group that does not deactivate the oxygen atom to which it is attached,
   optionally in an inert anhydrous organic medium, to form a corresponding compound of formula XII

   (E) - R₁₄O - CH=CH - CH=N⁺(R₁₂)R₁₃ Xₐ⁻ (XII)

   wherein R₁₂, R₁₃, R₁₄ and Xₐ are as defined above, and
(iii) hydrolyzing that compound of formula XII to obtain a corresponding compound of formula VII in free base or acid addition salt form and, if in acid addition salt form, neutralizing the acid addition salt with base.

In a variant of process A step (iii) may be dispensed with and a compound of formula XII used directly in, e.g., process B.

Steps (i) and (ii) may be carried out simultaneously or step (ii) may follow step (i); step (iii) follows step (ii) and step (iv) (see hereafter), when employed, follows step (iii).

R₁₂ is R₁₂ₐ, where R₁₂ₐ is C₁₋₃alkyl; or R_{12b}, where R_{12b} with the exception of C₁₋₃alkyl has the significance indicated above for R₁₂ (i.e. it is phenyl or phenyl substituted by 1 to 3 substituents each of which is independently C₁₋₃alkyl, C₁₋₃alkoxy, fluoro, chloro, bromo or nitro with a maximum of two nitro groups).

R₁₂ₐ is preferably C₁₋₂alkyl and most preferably methyl.

R_{12b} is preferably R_{12b}' where R_{12b}' is phenyl or phenyl substituted by 1 or 2 substituents each of which is independently C₁₋₃alkyl, C₁₋₂alkoxy or chloro, more preferably R_{12b}'' where R_{12b}'' is phenyl or phenyl substituted by 1 or 2 methyl groups, and most preferably phenyl.

R₁₃ is preferably C₁₋₂alkyl and most preferably methyl.

R₁₄ is preferably C₁₋₆alkyl, preferably primary or secondary C₂₋₄alkyl, more preferably n-C₂₋₄alkyl and most preferably ethyl or n-butyl.

Each Xₐ is preferably chloro or bromo, especially chloro. Each Xₐ⁻ is preferably chloride or bromide, especially chloride. The base utilized in the hydrolysis or neutralization of step (iii) is preferably an inorganic base such as sodium carbonate, potassium carbonate, sodium hydroxide or potassium hydroxide and more preferably is sodium carbonate or potassium carbonate.

Preferred reaction conditions for process A are as follows:
Step (i) [when carried out prior to Step (ii)]:
   - Temperature:: -20° to +50°C
   - Time:: 1.5 to 5 hours
   - Reaction medium:: liquid halogenated lower alkane, e.g. 1,2-dichloroethane and methylene chloride; or acetonitrile; methylene chloride and acetonitrile being most preferred
   - Molar ratio of reactants:: 1 to 1.5 moles IX per mole VIII
Step (ii) [when carried out subsequent to step (i)]:
   - Temperature:: 10° to 60°C, 10° to 40°C being more preferred
   - Time:: 0.5 to 3 hours
   - Reaction medium:: same as step (i)
   - Molar ratio of reactants:: 1 to 1.5 moles XI per mole VIII utilized in step (i)
Steps (i) and (ii) [when carried out simultaneously]:
   - Temperature:: -15° to +35°C
   - Time:: 2 to 6 hours
   - Reaction medium:: same as step (i) when carried out prior to step (ii)
   - Molar ratio of reactants:: 1 to 1.5 moles IX and 1 to 1.5 moles XI per mole VIII
Step (iii):
   - Temperature:: 0° to 65°C
   - Time:: 0.5 to 3 hours
   - Reaction medium:: water or mixture of water and reaction medium utilized in step (ii)
   - Molar ratio of reactants:: 2 to 4 equivalents base per mole IX utilized in step (i)

It is preferred to effect the hydrolysis of step (iii) with base.

The reaction medium for steps (i) and (ii) may, alternatively and preferably, consist of the neat reagents, i.e. the reagents in the absence of any solvent, i.e. for step (i) the compounds of formulae VIII and IX and for step (ii) the compounds of formulae X and XI. This is very advantageous from e.g. an ecological point of view since the presence of solvents such as acetonitrile in waste water or the emission of vapours of e.g.methylene chloride into the atmosphere is avoided thereby. The compounds of formulae VIII and IX or, respectively, X and XI can be brought to react in the absence of solvent because they do not form a solid block when mixed together but, surprisingly, form a suspension.

Process A may be divided into two subprocesses depending upon the significances of R₁₂ and R₁₃:
(1) R₁₂ and R₁₃ independently are C₁₋₃alkyl **(subprocess Aa)** and
(2) at least one of R₁₂ and R₁₃ is other than C₁₋₃alkyl
**(subprocess Ab)**.

The product of step (iii) of **subprocess Aa** often contains an appreciable amount of the compound of formula XIII

(E) - OHC - CH=CH - OR₁₄ (XIII)

wherein R₁₄ is as defined above,
corresponding to the obtained compound of formula VII, the molar ratio of the compound of formula VII to the compound of Formula XIII typically being about 2:1. While it is, of course, possible to separate the compound of formula VII from that of formula XIII by conventional means of separation, it is preferable to subject the product of step (iii), i.e. the crude compound of formula VII (a mixture of the compound of formula VII with the corresponding compound of formula XIII), to step (iv), i.e.:
(iv) treating the crude mixture containing the compound of formula VIIa

   (E) - OHC - CH=CH - N(R₁₂ₐ)R₁₃ (VIIa)

   wherein R₁₂ₐ and R₁₃ are as defined above,
   with a corresponding compound of formula XIV

   H - N(R₁₂ₐ)R₁₃ (XIV)

   wherein R₁₂ₐ and R₁₃ are as defined above,
   to convert any compound of formula XIII present therein into additional compound of formula VIIa.

Preferred reactants in subprocess Aa are those
(a) wherein R₁₂ₐ is C₁₋₂alkyl, R₁₃ is C₁₋₂alkyl, R₁₄ is primary or secondary C₂₋₄alkyl, each Xₐ is chloro, and each Xₐ⁻ is chloride;
(b) as.(a) but wherein R₁₄ is n-C₂₋₄alkyl;
(c) as (b) but wherein R₁₂ₐ is methyl, R₁₃ is methyl, and R₁₄ is ethyl;
(d)-(f) as (a)-(c) but wherein the base utilized in step (iii) is sodium carbonate or potassium carbonate; and
(g)-(i) as (d)-(f) but wherein the base utilized in step (iii) is potassium carbonate.

Preferred reaction conditions for subprocess Aa, particularly when the reactants are those of subgroups (a), (d) and (g), more particularly when they are those of subgroups (b), (e) and (h) and especially when they are those of subgroups (c), (f) and (i), are:
Step (i):
   - Temperature:: 0° to 20°C, 0° to 15°C being more preferred and 5° to 15°C being even more preferred
   - Time:: 1.5 to 4 hours
   - Reaction medium:: liquid halogenated lower alkane or acetonitrile, or the neat reagents; methylene chloride or the neat reagents being most preferred
   - Molar ratio of reactants:: 1 to 1.2 moles IX per mole VIII 1.1 to 1.2 moles IX per mole VIII being more preferred;
Step (ii):
   - Temperature:: 25° to 40°C
   - Time:: 0.7 to 2.5 hours
   - Reaction medium:: same as step (i)
   - Molar ratio of reactants:: 1 to 1.2 moles XI per mole VIII utilized in step (i), 1.1 to 1.2 moles IX per mole VIII being more preferred;
Step (iii):
   - Temperature:: 20° to 65°C, 20° to 30°C being more preferred
   - Time:: 0.75 to 2 hours
   - Reaction medium:: aqueous
   - Molar ratio of reactants:: 2 to 4 equivalents base per mole IX utilized in step (i);
Step (iv):
   - Temperature:: 0° to 20°C, 10° to 20°C being more preferred
   - Time:: 0.3 to 1 hour
   - Reaction medium:: C₁₋₄alkanol, methanol being most preferred
   - Molar ratio of reactants:: 0.15 to 1 mole XIV per mole VIII utilized in step (i), 0.15 to 0.4 mole XIV per mole VIII being more preferred.
In **subprocess Aa** step (ii) is preferably carried out after step (i).

Preferably, subprocess Aa comprises
(i) reacting N,N-dimethylformamide (compound of formula VIII wherein R₁₂ and R₁₃ are methyl) with oxalyl chloride (compound of formula IX wherein Xₐ is chloro) neat or in methylene chloride at a temperature of 0° to 15°C to form the compound of formula Xa

   Cl - CH=N⁺(CH₃)₂ Cl⁻ (Xa)
(ii) reacting the compound of formula Xa with ethyl vinyl ether (compound of formula XI wherein R₁₄ is ethyl) neat or in methylene chloride at a temperature of 25° to 40°C to form the compound of formula XIIa

   (E) - C₂H₅O - CH=CH-CH=N⁺(CH₃)₂ Cl⁻ (XIIa)
(iii) hydrolyzing the compound of formula XIIa with potassium carbonate in an aqueous medium at a temperature of 20° to 30°C to form a mixture of the compounds of formula VIIaa

   (E) - OHC - CH=CH - N(CH₃)₂ (VIIaa)

   and of formula XIIIa

   (E) - OHC - CH=CH - OC₂H₅ (XIIIa)
(iv) treating the mixture of compounds of formulae VIIaa and XIIIa with dimethylamine (compound of formula XIV wherein R₁₂ₐ and R₁₃ are methyl) in methanol at a temperature of 10° to 20°C to convert the compound of formula XIIIa into additional compound of formula VIIaa.

More preferably, in subprocess Aa
(1) the molar ratio of oxalyl chloride to N,N-dimethylformamide in step (i) is from 1:1 to 1.2:1, and step (i) is carried out by adding oxalyl chloride to N,N-dimethylformamide neat or in solution in methylene chloride over a period of 1.5 to 4 hours at a rate such that the temperature is maintained at 5° to 15°C;
(2) in step (ii) the molar ratio of ethyl vinyl ether to the N,N-dimethylformamide utilized in step (i) is from 1:1 to 1.2:1, and step (ii) is carried out by adding ethyl vinyl ether to the reaction mixture over a period of 0.4 to 1.5 hours at a rate such that the temperature does not exceed 30°C and, upon completion of the addition, refluxing the reaction mixture at 35° to 40°C for 0.3 to 1 hour and if indicated recovering as much methylene chloride as possible at a temperature not in excess of 45°C;
(3) in step (iii) the molar ratio of potassium carbonate to the oxalyl chloride utilized in step (i) is from 1:1 to 2:1, and step (iii) is carried out by adding water to the product of step (ii) stirred at 20° to 30°C, allowing the temperature to rise to 45° to 60°C, maintaining this temperature during the balance of the addition of the water and for an additional 0.3 to 1 hour, cooling the reaction mixture to 15° to 25°C, adding an aqueous solution of potassium carbonate over a period of 0.3 to 1.25 hours at this temperature, extracting the mixture with methylene chloride and distilling as much methylene chloride as possible at a temperature not in excess of 45°C; and
(4) in step (iv) the molar ratio of dimethylamine to the N,N-dimethylformamide utilized in step (i) is from 0.15:1 to 0.4:1, and step (iv) is carried out by adding anhydrous dimethylamine to a solution of the product of step (iii) in methanol stirred at 10° to 20°C at a rate such that the temperature does not exceed 20°C and distilling the solvent and any excess dimethylamine at a temperature not in excess of 120°C.

Preferred reactants in subprocess Ab are those
(a) wherein R_{12b} is R_{12b}', R₁₃ is C₁₋₂alkyl, R₁₄ is primary or secondary C₂₋₄alkyl, each Xₐ is chloro, and each Xₐ⁻ is chloride;
(b) as. (a) but wherein R_{12b} is R_{12b}'', and R₁₄ is n-C₂₋₄alkyl;
(c) as (b) but wherein R_{12b} is phenyl, R₁₃ is methyl, and R₁₄ is ethyl or n-butyl, especially n-butyl;
(d)-(f) as (a)-(c) but wherein the base utilized in step (iii) is sodium carbonate or potassium carbonate, and
(g)-(i) as (d)-(f) but wherein the base utilized in step (iii) is sodium carbonate.

Preferred reaction conditions for subprocess Ab, particularly when the reactants are those of subgroups (a), (d) and (g), more particularly when they are those of subgroups (b), (e) and (h) and especially when they are those of subgroups (c), (f) and (i), are:
Step (i) [when carried out prior to step (ii)]:
   - Temperature:: -20° to +45°C
   - Time:: 1.5 to 5 hours
   - Reaction medium:: liquid halogenated lower alkane or acetonitrile, methylene chloride and acetonitrile being more preferred and acetonitrile being most preferred; or, most preferably, the neat reagents;
   - Molar ratio of reactants:: 1 to 1.2 moles IX per mole VIII, 1.1 to 1.2 moles IX per mole VIII being more preferred;
Step (ii) [when carried out subsequent to step (i)]:
   - Temperature:: 10° to 40°C
   - Time:: 0.5 to 3 hours
   - Reaction medium:: same as step (i)
   - Molar ratio of reactants:: 1 to 1.3 moles XI per mole VIII utilized in step (i), 1.1 to 1.25 moles XI per mole VIII being more preferred;
Steps (i) and (ii) [when carried out simultaneously]:
   - Temperature:: -15° to +35°C
   - Time:: 2 to 6 hours
   - Reaction medium:: same as step (i) when carried out prior to step (ii)
   - Molar ratio of reactants:: 1 to 1.5 moles IX and 1 to 1.5 moles XI per mole VIII;
Step (iii):
   - Temperature:: 0° to 35°C, 0° to 30°C being more preferred
   - Time:: 0.5 to 1.5 hours
   - Reaction medium:: mixture of water and reaction medium of step (ii)
   - Molar ratio of reactants:: 2 to 4 equivalents base per mole IX utilized in step (i).

There are three preferred variants of **subprocess Ab**, namely **variants Ab1, Ab2 and Ab3**. In variant Ab1 R₁₄ is ethyl and the reaction medium for steps (i) and (ii) is methylene chloride or, preferably, the neat reagents, and in variants Ab2 and Ab3 R₁₄ is n-butyl and the reaction medium for steps (i) and (ii) is acetonitrile or, preferably, the neat reagents. In variants Ab1 and Ab2, step (ii) is carried out after step (i) and in variant Ab3 steps (i) and (ii) are carried out simultaneously; in each variant, step (iii) follows steps (i) and (ii).

**Variant Ab1 of subprocess Ab** preferably comprises
(i) reacting N-methylformanilide (compound of formula VIII wherein R₁₂ is phenyl and R₁₃ is methyl) with oxalyl chloride (compound of formula IX wherein Xₐ is chloro) neat or in methylene chloride at a temperature of 15° to 45°C to form the compound of formula Xb

   Cl - CH=N⁺(C₆H₅)CH₃ Cl⁻ (Xb)
(ii) reacting the compound of formula Xb with ethyl vinyl ether (compound of formula XI wherein R₁₄ is ethyl) neat or in methylene chloride at a temperature of 15° to 40°C to form the compound of formula XIIb1

   (E) - C₂H₅O - CH=CH-CH=N⁺(C₆H₅)CH₃ Cl⁻ (XIIb1)
(iii) hydrolyzing the compound of formula XIIb1 with sodium carbonate in a mixture of methylene chloride and water at a temperature of 20° to 30°C to obtain the compound of formula VIIb

   (E) - OHC - CH=CH-N(C₆H₅)CH₃ (VIIb)

More preferably, in variant Ab1 of subprocess Ab,
(1) the molar ratio of oxalyl chloride to N-methylformanilide in step (i) is from 1:1 to 1.2:1, and step (i) is carried out by adding oxalyl chloride to N-methylformanilide neat or in solution in methylene chloride at 15° to 20°C over a period of 1 to 2 hours and, upon completion of the addition, gradually raising the temperature of the reaction mixture to 40° to 45°C over a period of 0.75 to 1.25 hours and then refluxing it for 0.75 to 1.25 hours;
(2) in step (ii) the molar ratio of ethyl vinyl ether to the N-methylformanilide utilized in step (i) is 1 to 1.3:1, and step (ii) is carried out by cooling the product of step (i) to 15° to 20°C, adding ethyl vinyl ether over a period of 0.5 to 1.5 hours at a rate such that the temperature does not exceed 30°C, and, upon completion of the addition, refluxing the reaction mixture for 0.3 to 0.7 hour; and
(3) in step (iii) the molar ratio of sodium carbonate to the oxalyl chloride utilized in step (i) is from 1:1 to 1.2:1, and step (iii) is carried out by cooling the product of step (ii) to 15° to 20°C, adding, over a period of 0.5 to 1 hour, an aqueous solution of sodium carbonate at a rate such that the temperature of the reaction mixture is 20° to 30°C and, upon completion of the addition, stirring the mixture at 20° to 30°C for 0.2 to 0.5 hour, allowing the mixture to separate into two phases, separating the two phases and recovering the product from the organic phase.

**Variant Ab2 of subprocess Ab** preferably comprises
(i) reacting N-methylformanilide with oxalyl chloride neat or in acetonitrile at a temperature of from -20° to +20°C to form the compound of formula Xb
(ii) reacting the compound of formula Xb with n-butyl vinyl ether (compound of formula XI wherein R₁₄ is n-butyl) neat or in acetonitrile at a temperature of 10° to 40°C to form the compound of formula XIIb2

   (E) - n-C₄H₉O - CH=CH-CH=N⁺(C₆H₅)CH₃ Cl⁻ (XIIb2)

   and
(iii) hydrolyzing the compound of formula XIIb2 with sodium carbonate in a mixture of acetonitrile and water at a temperature of 0° to 25°C to obtain the compound of formula VIIb.

More preferably, in variant Ab2 of subprocess Ab,
(1) the molar ratio of oxalyl chloride to N-methylformanilide in step (i) is from 1:1 to 1.2:1, and step (i) is carried out by adding oxalyl chloride to N-methylformanilide neat or in solution in acetonitrile at -18° to +8°C over a period of 1 to 2 hours and, upon completion of the addition, gradually raising the temperature of the reaction mixture to 12° to 20°C over a period of 0.4 to 0.75 hour and then stirring it for 0.2 to 0.4 hour at this temperature;
(2) in step (ii) the molar ratio of n-butyl vinyl ether to the N-methylformanilide utilized in step (i) is from 1:1 to 1.2:1, and step (ii) is carried out by adding n-butyl vinyl ether to the product of step (i) stirred at 12° to 20°C over a period of 0.5 to 1.5 hours at a rate such that the temperature does not exceed 30°C, and, upon completion of the addition, stirring the reaction mixture for 0.3 to 0.7 hour at 25° to 35°C; and
(3) in step (iii) the molar ratio of sodium carbonate to the oxalyl chloride utilized in step (i) is from 1:1 to 1.3:1, and step (iii) is carried out by cooling the product of step (ii) to 0° to 5°C, adding, over a period of 0.5 to 1.2 hours, an aqueous solution of sodium carbonate at a rate such that the temperature of the reaction mixture is 5° to 12°C and, upon completion of the addition, adding toluene, stirring the mixture at 15° to 25°C for 0.2 to 0.5 hour, allowing the mixture to separate into two phases, separating the two phases and recovering the product from the organic phase.

**Variant Ab3 of subprocess Ab** preferably comprises(i) and (ii) reacting N-methylformanilide with oxalyl chloride neat or in
acetonitrile at -10° to +30°C in the presence of n-butyl vinyl ether to form the compound of formula Xb,
which compound then reacts with the n-butyl vinyl ether in the reaction mixture to form the compound of formula XIIb2, and
(iii) hydrolyzing the compound of formula XIIb2 with sodium carbonate in a mixture of acetonitrile and water at a temperature of 0° to 25°C to obtain the compound of formula VIIb.

More preferably, in variant Ab3 of subprocess Ab,
(1) in steps (i) and (ii), the molar ratio of each of oxalyl chloride and n-butyl vinyl ether to N-methylformanilide is from 1:1 to 1.2:1, and steps (i) and (ii) are carried out by adding a solution of N-methylformanilide and n-butyl vinyl ether neat or in acetonitrile to oxalyl chloride neat or in solution in acetonitrile stirred at -10° to +10°C over a period of 2 to 3 hours and, upon completion of the addition, gradually raising the temperature of the reaction mixture to 20 to 30°C over a period of 0.4 to 1.5 hours and then stirring the reaction mixture at this temperature for 0.5 to 1.5 hours; and
(2) in step (iii), the molar ratio of sodium carbonate to the oxalyl chloride utilized in step (i) is from 1:1 to 1.3:1, and step (iii) is carried out by cooling the product of step (ii) to 0° to 5°C, adding, over a period of 0.5 to 1.2 hours, an aqueous solution of sodium carbonate at a rate such that the temperature of the reaction mixture is 0° to 12°C and, upon completion of the addition, adding toluene, stirring the mixture at 15° to 25°C for 0.2 to 0.5 hour, allowing the mixture to separate into two phases, separating the two phases and recovering the product from the organic phase.

The product of step (iii) of subprocess Ab may be subjected to a step (iv) analogous to step (iv) of subprocess Aa. However, there is usually no reason to do so since the product usually contains little or no compound of formula XIII.

### 7.2. Process B (preparation of the compounds of formula Va from the compounds of formula VIIc)

The process for the preparation of the compounds of formula Va **(process B)** comprises
(i) reacting a compound of formula VIIc

   (E) - OHC - CH=CH-N(R_{12b})R₁₃ (VIIc)

   wherein R_{12b} and R₁₃ are as defined above,
   with a compound of formula XV

   PO(X_{b})₃ (XV)

   wherein X_{b} is chloro or bromo, or
   with a compound selected from oxalyl chloride or bromide; phosgene or carbonyl bromide; phosphorus trichloride or tribromide; phosphorus pentachloride or pentabromide; and an alkyl- or arylsulfonyl chloride or bromide, such as p-toluenesulfonyl chloride or bromide or methanesulfonyl chloride or bromide;
   to form the corresponding compound of formula XVI

   (E) - X_{b} - CH=CH-CH=N⁺(R_{12b})R₁₃ (XVI)

   and the corresponding anion, e.g.⁻PO₂(X_{b})₂,
   wherein X_{b}, R_{12b} and R₁₃ are as defined above,
(ii) reacting that compound of formula XVI
   with a compound of formula XVII 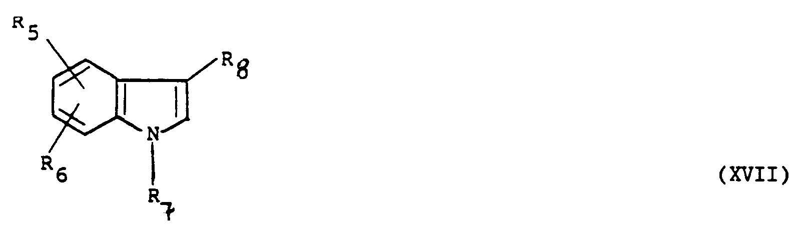 wherein R₅, R₆, R₇ and R₈ are as defined above,
   to form a corresponding compound of formula XVIII 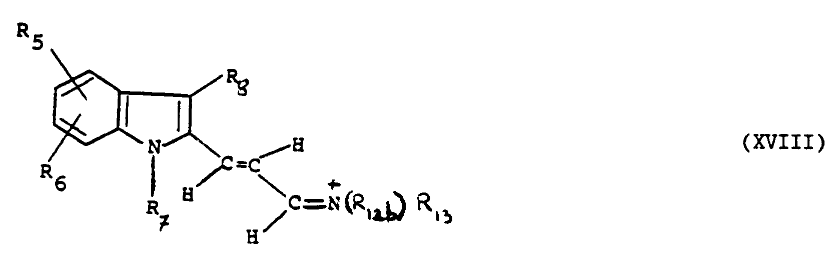 and the corresponding anion, e.g. ⁻PO₂(X_{b})₂,
   wherein R₅, R₆, R₇, R₈, R_{12b}, R₁₃ and X_{b} are as defined above, and
(iii) hydrolyzing that compound of formula XVIII to obtain a corresponding compound of formula Va.

As mentioned above for process A, in a variant, in step (i) a compound of formula XII obtained according to process A may be used directly in place of a compound of formula VIIc.

The preferred significances for R_{12b} and R₁₃ are set forth above, and the preferences for R₅, R₆, R₇ and R₈ are those set forth for R₀, R, R₂ and R₃, respectively, in USP 4 739 073. X_{b} preferably is chloro.

A compound of formula VIIc preferably is reacted with a compound of formula XV.

Steps (i) and (ii) preferably are effected in an inert anhydrous organic medium.

Preferred reactants (and final products) are
(a)-(d) those wherein R_{12b} is R_{12b}', R₁₃ is C₁₋₂alkyl, each X_{b} is chloro, and R₅ to R₈ have the significances of the corresponding variables of Groups (i), (ii), (xxi) and (xxii) of USP 4 739 073;
(e)-(h) those of (a)-(d) wherein R_{12b} is R_{12b}'', and R₅ to R₈ have the significances of the corresponding variables of Groups (v), (vi), (xxv) and (xxvi) of USP 4 739 073;
(i) and (j) those of (e) and (f) wherein R₇ is C₁₋₃alkyl, R₈ is phenyl, methylphenyl, fluorophenyl, dimethylphenyl or methyl-fluorophenyl, R₅ is hydrogen, C₁₋₃alkyl or 4- or 6-benzyloxy, and R₆ is hydrogen or methyl;
(k) and (l) those of (g) and (h) wherein R₇ is phenyl, methylphenyl, fluorophenyl, dimethylphenyl or methylfluorophenyl, R₈ is C₁₋₃alkyl, R₅ is hydrogen, C₁₋₃alkyl or 4- or 6-benzyloxy, and R₆ is hydrogen or methyl;
(m)-(p) those of (i)-(l) wherein R₅ is hydrogen, and R₆ is hydrogen;
(q)-(t) those of (m)-(p) wherein R_{12b} is phenyl, and R₁₃ is methyl;
(u) that of (q) wherein R₇ is 1-methylethyl, and R₈ is 4-fluorophenyl; and
(v) that of (s) wherein R₇ is 4-fluorophenyl, and R₈ is 1-methylethyl.

Most preferably, R₅ and R₆ are hydrogen, R₇ is 1-methylethyl and R₈ is para-fluorophenyl.

The compounds of formula VIIc used for step (i) may be in free base form or, preferably, in acid addition salt form, e.g. in hydrochloride acid addition salt form.

The preferred bases for step (iii) are inorganic hydroxides such as sodium hydroxide and potassium hydroxide, especially the former. However, as set forth infra, it is most preferred not to employ any base in step (iii).

Preferred reaction conditions for process B are:
Step (i):
   - Temperature:: -10° to +25°C, -10° to +10°C being more preferred
   - Time:: 0.1 to 1.2 hours, 0.5 to 1 hour being more preferred
   - Reaction medium:: lower alkyl nitrile, acetonitrile being most preferred
   - Molar ratio of reactants:: 1 to 1.5 moles XV per mole VIIc, 1.1 to 1.3 moles XV per mole VIIc being more preferred
Step (ii):
   - Temperature:: 60° to 100°C, 65° to 85°C being more preferred
   - Time:: 2 to 30 hours, 3 to 24 hours being more preferred
   - Reaction medium:: same as step (i)
   - Molar ratio of reactants:: 1 to 5 moles XVI per mole XVII, 2 to 3 moles XVI per mole XVII being more preferred (100% yield in step (i) assumed in each case)
Step (iii):
   - Temperature:: 10° to 40°C when base is employed and 35° to 60°C when it is not
   - Time:: 0.1 to 1 hour when base is employed and 2 to 4 hours when it is not
   - Solvent:: mixture of water and reaction medium of step (ii)
   - Molar ratio of reactants:: when base is employed, 4 to 8 equivalents base, preferably sodium hydroxide or potassium hydroxide, per mole XV utilized in step (i).

Even more preferred reaction conditions for process B, particularly when the reactants and final products are those of subgroups (a)-(v), especially of subgroups (i), (j), (m), (n), (q), (r) and (u), are:
Step (i):
   - Temperature:: -10° to +10°C
   - Time:: 0.75 to 1 hour
   - Reaction medium:: acetonitrile
   - Molar ratio of reactants:: 1.1 to 1.3 moles XV per mole VIIc
Step (ii):
   - Temperature:: 65° to 85°C, 80° to 83°C being more preferred
   - Time:: 3 to 16 hours, 3 to 10 hours being more preferred
   - Reaction medium:: acetonitrile
   - Molar ratio of reactants:: 2 to 3 moles XVI per mole XVII, 2.1 to 2.5 moles XVI per mole XVII being more preferred (100% yield in step (i) assumed in each case)
Step (iii):
   - Temperature:: 20° to 55°C; 25° to 35°C being preferred when base is employed, and 35° to 55°C when it is not
   - Time:: 0.3 to 0.7 hour when base is employed and 2 to 3 hours when it is not
   - Reaction medium:: mixture of water and reaction medium of step (ii)
   - Molar ratio of reactants:: when base is employed, 4 to 6 equivalents base, preferably sodium hydroxide, per mole XV utilized in step (i).

It is most preferred not to employ base in step (iii).

### 8. General conditions applicable to all processes

Most of the molar amounts (ratios) recited herein are merely exemplary and may be varied, as is evident to one of ordinary skill in the art.

Steps (i) and (ii) of process A (including subprocesses Aa and Ab and the variants thereof), steps (i) and (ii) of process B and, preferably, step (iv) of subprocess Aa are preferably carried out under anhydrous conditions and an inert atmosphere, preferably dry helium, argon or nitrogen, or a mixture thereof, usually dry nitrogen. Step (iii) of process A (including subprocesses Aa and Ab and the variants thereof) and process B are often, but need not be, carried out under an inert atmosphere.

Likewise, most of the temperature ranges given above are merely exemplary. All temperatures are internal temperatures, unless otherwise indicated. As utilized above, the term "reaction medium" embraces mixtures of liquids and implies that the reaction medium is a liquid at the desired reaction temperature. It should, therefore, be understood that not all of the liquids listed for a particular step may be utilized for the entire recited temperature range. It should also be understood that the reaction medium must be at least substantially inert to the reactants employed, intermediates generated and end products under the reaction conditions utilized. It should be understood that the reaction temperature may exceed the boiling point of a reactant or the reaction medium if a condenser or a closed system (reaction bomb) is utilized.

The reaction times set forth above are also merely exemplary and may be varied.

It will also be understood that conventional work-up procedures may be employed. The term "solvent", as utilized herein, embraces mixtures of solvents and implies that the reaction medium is a liquid at the desired reaction temperature. Unless indicated otherwise all solvent mixtures are by volume. The term "inert atmosphere" means an atmosphere that does not react with any of the reactants, intermediates or end products or otherwise interfere with the reaction.

The product of each process may, if desired, be purified by conventional techniques such as recrystallization, chromatography or fractional distillation.

All temperatures are in degrees Centigrade and room temperature is 20° to 30°C, usually 20° to 25°C unless otherwise indicated; evaporations are done under vacuum employing minimal heating, drying of organic phases is done over anhydrous magnesium sulfate, and unless otherwise indicated, silica gel is utilized for all column chromatographies.

It will be appreciated that the process variants disclosed with the present invention are improvements over known similar processes; they may be used to obtain the desired end products, i.e. 7-substituted-hept-6-enoic and -heptanoic acids and derivatives thereof, e.g. more easily, or in e.g. a greater state of chemical or optical purity than can be achieved with conventional methods.

### 9. Specific embodiment

A specific illustration of the general inventive concept underlying the above process variants concerns the preparation of erythro-(E)-3,5-dihydroxy-7-[3'-(4''fluorophenyl)-1'-(1''-methylethyl)indol-2'-yl]-hept-6-enoic acid of formula Ia 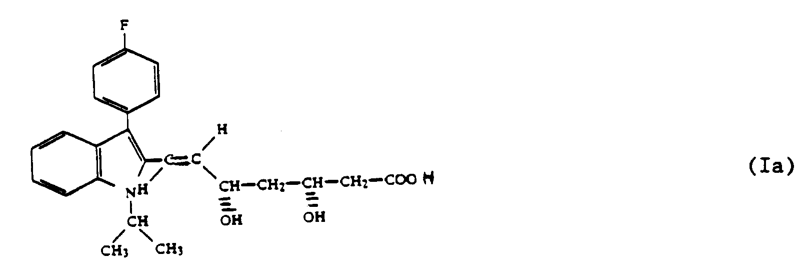
in racemic or optically pure form; in free acid, salt, ester or δ-lactone, i.e. internal ester, form, in a multistep process using all the above process variants, namely processes A, B and the stereoselective reduction of a compound of formula II, and comprising:
- according to process A:
   a) reacting a compound of formula VIIIa

      OHC - N(R_{12b})R₁₃ (VIIIa)

      wherein R_{12b} and R₁₃ are as defined above,
      with a compound of formula IX, optionally in an inert anhydrous organic medium,
      to form a corresponding compound of formula Xc

      Xₐ - CH=N⁺(R_{12b})R₁₃ Xₐ⁻ (Xc)

      wherein Xₐ, R_{12b} and R₁₃ are as defined above;
   b) reacting that compound of formula Xc with a compound of formula XI, optionally in a inert anhydrous organic medium, to form a corresponding compound of formula XIIc

      (E) - R₁₄O - CH=CH-CH=N⁺(R_{12b})R₁₃ Xₐ⁻ (XIIc)

      wherein R_{12b}, R₁₃, R₁₄ and Xₐ are as defined above;
   c) hydrolyzing that compound of formula XIIc to obtain a corresponding compound of formula VIIc in free base or acid addition salt form and, if in acid addition salt form, neutralizing the acid addition salt with base;
- according to process B:
   d) reacting that compound of formula VIIc
      with a compound of formula XV or
      with a compound selected from oxalyl chloride or bromide; phosgene or carbonyl bromide; phosphorus trichloride or tribromide; phosphorus pentachloride or pentabromide; and an alkyl- or arylsulfonyl chloride or bromide, such as p-toluenesulfonyl chloride or bromide or methanesulfonyl chloride or bromide;
      to form a corresponding compound of formula XVI and the corresponding anion, e.g. ⁻PO₂(X_{b})₂;
   e) reacting that compound of formula XVI with 3-(4'-fluorophenyl)-1-(1'-methylethyl)-1H-indole (the compound of formula XVII wherein R₅ and R₆ are hydrogen, R₇ is 1-methylethyl and R₈ is p-fluorophenyl) to form a corresponding compound of formula XVIIIa 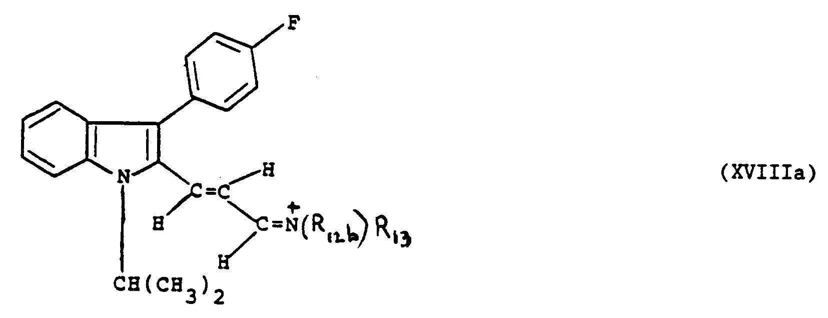 and the corresponding anion, e.g. ⁻PO₂(X_{b})₂,
      wherein R_{12b}, R₁₃ and Xₐ are as defined above;
   f) hydrolyzing that compound of formula XVIIIa to obtain (E)-3-[3'-(4''-fluorophenyl)-1-(1''-methylethyl)-1H-indol-2'-yl]-prop-2-enal (the compound of formula Va wherein R₅ and R₆ are hydrogen, R₇ is 1-methylethyl and R₈ is p-fluorophenyl);
   g) reacting that compound of formula Va with the dianion of an acetoacetic ester of formula CH₃COCH₂COOR₁ wherein R₁ is as defined above,
      to obtain a corresponding compound of formula IIa 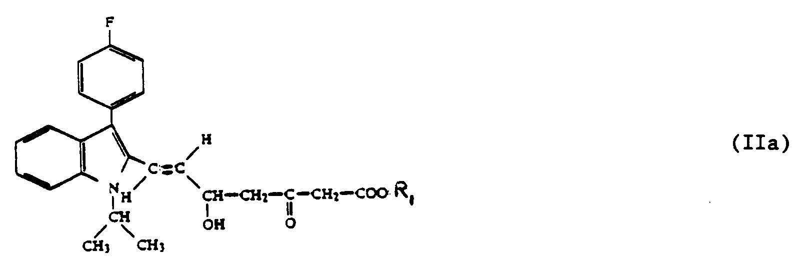 wherein R₁ is as defined above; in racemic or optically pure form;
- according to the stereoselective reduction process:
   h) stereoselectively reducing the racemic or optically pure compound of formula IIa by,
      in a **first step** [= step (a) under 5. above], mixing a compound of formula III with sodium borohydride (NaBH₄) in a reaction medium comprising an alcohol and tetrahydrofuran;
      in a **second step** [= step (b) under 5. above], treating a compound of formula IIa in racemic or optically pure form with the resultant mixture under conditions suitable to obtain a mixture containing a cyclic boronate compound of formula IV(a) and/or a boron complex of formula IV(b) wherein
      R is [3-(4'-fluorophenyl)-1-(1'-methylethyl)-1H-indol]-2-yl,
      X is -CH=CH- and
      R₁ and R₃ are as defined above; and
      in a **third step** [= step (c) under 5. above], cleaving the product obtained in the second step to obtain the compound of formula Ia in ester form; in racemic or optically pure form; and
   i) if desired, converting the compound of formula Ia in ester form by conventional means into the free acid form, a salt form, a further ester form or the δ-lactone, i.e. internal ester, form.

The compound of formula Ia may be in free acid, salt, ester or δ-lactone, i.e. internal ester, form. It preferably is in free acid or salt, preferably alkaline salt, especially sodium salt form. It preferably is in racemic or, alternatively, optically pure (3R,5S) enantiomeric form it especially is in racemic form. As appears from formula Ia that form is the erythro form.

It will be appreciated that the specific embodiment illustrated under 9. is effected either in accordance with the procedures disclosed with this invention or, for some of the steps, in accordance with procedures known in the art.

Thus
- steps a), b) and c) are effected as described above under 7.1. for subprocess Ab, especially steps i), ii) and, respectively, iii) thereof, e.g. according to variants Ab1, Ab2 and/or Ab3 of subprocess Ab; steps a) and b) may thus e.g. be carried out simultaneously as described above for process A:
- steps d), e) and f) are effected as described above under 7.2. for process B, especially steps i), ii) and, respectively, iii) thereof;
- step g) is effected according to procedures published, e.g. in USP 4 739 073, especially in Reaction Scheme I on column 8 and in Example 5, Step 5 on column 47 thereof;
- step h) is effected as described above under 5.;
- step i) is effected in conventional manner, e.g. as described in USP 4 739 073, especially in Reaction Schemes I (Reactions C, D and E) on column 9; Reaction Scheme II (Reaction L) on column 11; Reaction Scheme VIII (Reaction EE) on column 16; and in Examples 6(a), 6(b), 6(c), 8 and 9 on columns 49 and 50 and 52 and 53 thereof, whereby THF may advantageously be replaced by ethanol.

In the second part of the above stereoselective reduction step h), preferably a compound of formula IIa is used wherein R₁ is isopropyl or, especially, t-butyl, which facilitates the isolation of a relatively more pure compound of formula I than with a group R₁ such as methyl. Further, surprisingly, the compound of formula I obtained thereby is completely colourless, whereas it has always been obtained pale yellow in earlier syntheses.

As mentioned previously, the stereoselective reduction according to step h) above may be effected with a racemic or an optically pure compound of formula IIa. An optically pure compound of formula IIa is obtained e.g. by chromatographic resolution of a racemic compound of formula IIa obtained in step g) or, preferably, by an asymmetrical synthesis. Alternatively, resolution may be effected on a subsequent step, or on the racemic end product.

The starting materials for this specific embodiment of the invention are also known or may be prepared in accordance with known procedures. The preparation of 3-(4'-fluorophenyl)-1-(1'methylethyl)-1H-indole [a compound of formula XVII, see step e) above] is disclosed in USP 4 739 073 as Example 5, Steps 1 to 3 on columns 44 and 45, starting from fluorobenzene.

### 10. Examples

The following Examples are illustrative of the invention. All temperatures are in degrees Centigrade. The optical purity is expressed in percentage terms and thus e.g. "99.9 % pure erythro isomer" means that there is at most 0.9 % threo form in the compound obtained.

### 10.1. Examples for the stereoselective reduction of a compound of formula II to obtain a compound of formula I

### Example l: (+)-Erythro-(E)-7-[3'-(4''-fluorophenyl)-1'-(1''-methylethyl)indol-2'-yl]-3,5-dihydroxyhept-6-enoic acid tert-butyl ester

### [Formula I: R=3-(4'-fluorophenyl)-1-(1'-methylethyl)indol-2-yl; X = (E)-CH=CH-; R₁ = t-butyl; in racemic form]

(a) 47.67 g (1.26 moles) of sodium borohydride are added to a solvent comprising l.32 l of dry tetrahydrofuran (THF) and 356 ml of methanol under nitrogen at about -77°. To the resulting solution is added 102 ml of 50% (4.09 M) diethylmethoxyborane in THF over a l5 minute period, and the formed mixture is stirred for an additional 10 minutes.
(b) 300.5 g (0.464 mole) of 71.88 % pure (+)-(E)-7-[3'-(4''-fluorophenyl)-1'-(1''methylethyl)indol-2'yl]-5-hydroxy-3-oxohept-6-enoic acid tert-butyl ester in l04 ml THF and 26 ml of methanol at a temperature of from about -74° to -77°C are added dropwise over to the mixture formed in (a) over a period of l.5 hours, and the resulting mixture is stirred for an additional 30 minutes. 720 ml of saturated sodium bicarbonate solution and l.75 l of heptane are added to quench the reaction. 500 ml of ethyl acetate is then added and the resulting mixture is diluted with 3.5 l of water with stirring for l5 minutes, the temperature of the mixture being about l0°. The top organic layer is separated and washed several times with a total of 2.4 l of saturated sodium chloride solution, pH 7.5, and the organic layer is concentrated at 20-30 mm Hg at a maximum external temperature of about 45°. To the organic residue is added 375 ml of toluene, and the solvent is distilled at 20-30 mm Hg at a maximum external temperature of about 45°.
(c) 3.73 l of ethyl acetate is added to the obtained thick oil (the predominantly cyclic boronate). 500 ml of 30% hydrogen peroxide solution (4.41 moles) is then added to the ethyl acetate solution while maintaining an internal temperature of from 25 to 30° (the addition initially being exothermic), and the reaction mixture is stirred at 20 to 25° for about 2 hours until thin layer chromatography shows no boronate present. The top organic layer is washed twice with a total of 2.22 l of saturated sodium chloride solution, pH 7.5. The top organic layer is then separated, washed three times with a total of 2.6l l of 10% sodium sulfite solution (until the organic layer is free of peroxide) while maintaining an internal temperature of 25°. The top organic layer is then washed twice with a total of l.72 l of saturated sodium chloride solution, pH 7.5 and the solvent is distilled at 20-30 mm Hg and a maximum external temperature of about 45°. The residue is dissolved in l.17 l of refluxing ethyl acetate, the mixture is filtered while hot, and the filtrate is stirred at 20 to 25° for l8 hours. The solids are collected by filtration, dried under reduced pressure (about 20-30 mm Hg) at 25°C, washed with 550 ml of ethyl acetate/heptane (l:4), redissolved in 880 ml of ethyl acetate and stirred at ambient temperature for l8 hours. The solids are collected by filtration and washed with 480 ml of ethyl acetate/heptane (l:2). The solids are dried under reduced pressure to give a product of ll4.5 g (M.P. 135-137°).

A second crop is obtained from the mother liquors, to give a total yield of l49.5 g. The product has a chemical purity of 99.44% and is 99.67% pure erythro isomer. It may be resolved into two optically active enantiomers, the 3R, 5S and 3S,5R, of which the former is preferred.

Alternatively and preferably in step (a) one half of the indicated amount of sodium borohydride may be used.

Alternatively in step (c) aqueous sodium perborate solution may be used in place of 30 % hydrogen peroxide solution.

### Example 2: (+)-Erythro-(E)-7-[3'-(4''-fluorophenyl)-l'-(l''-methylethyl)-indol-2'-yl]-3,5-dihydroxyhept-6-enoic acid methyl ester

### [Formula I: R, X = as for Example 1; R₁ = methyl; in racemic form]

(a) Sodium borohydride is treated in a manner analogous to Example 1, step (a), but using 15 % diethylmethoxyborane in THF.
(b) 118.5 g (0.28 mole) of (+)-(E)-[7-(3'-(4''-fluorophenyl)-1'-(1''-methylethyl)indol-2'-yl]-5-hydroxy-3-oxohept-6-enoic acid methyl ester is treated analogously to Example 1, step (b) but dilution is effected with 1.42 l of water and l.l85 l of heptane, instead of 3.5 l of water alone.
(c) To the organic residue (the predominantly cyclic boronate) is added 2.375 l of ethyl acetate and the mixture is treated with 264 ml of 30 % hydrogen peroxide solution (2.328 moles) and worked up as described in Example 1, step (c). The residue is then dissolved in l30 ml of isopropanol. The mixture is heated to refluxing temperature. While hot, l4 g of boric are added and refluxing is continued for 15 minutes. The mixture is then filtered and the filtrate is stirred at 20° to 25° for l8 hours. The solids are collected by filtration, washed with l00 ml of isopropanol, and dried under reduced pressure to give a product of ll0 g (80 % yield). The product is redissolved in methanol and recrystallized [M.P. 124-126°]. The product is 99.07 % pure erythro racemate, which may be resolved into two optical enantiomers, the 3R,5S and 3S,5R, of which the former is preferred.

### Example 3: (+)-Erythro(E)-3,5-dihydroxy-7-[l'-(4''-fluorophenyl)--4'-(l''-methylethyl)-2'-phenyl-lH-imidazol-5'-yl]-hept-6-enoic acid tert-butyl ester

### [Formula I: R a 1-(4'-fluorophenyl)-4-(1'-methylethyl)-2-phenyl-1H-imidazol-5-yl; X = (E)-CH=CH-; R₁ = tert-butyl; in (3R,5S)-enantiomeric form]

(a) 10.27 g (0.27 mole) of sodium borohydride are added to a solvent comprising 1.67 liter of dry THF and 513 ml of methanol under nitrogen at about -76°. To the resulting solution is added 387 ml of 15 % diethylmethoxyborane in THF over a 30 minute period, while maintaining the internal temperature below -77.5°, and the formed mixture is stirred for an additional 5 minutes.
(b) 110 g (0.223 mole) of (5S)(E)-7-[l'-(4''-fluorophenyl)-4'-(l''-methylethyl)-2'-phenyl-lH-imidazol-5'-yl]-5-hydroxy-3-oxohept-6-enoic acid tert-butyl ester in 304 ml of THF and 76 ml of methanol at a temperature of about -74° to -77° are added dropwise to the mixture formed in (a) over a period of two hours. The resulting yellow solution is stirred at -76.5° for six hours. 425 ml of saturated ammonium chloride is then added to quench the reaction, the temperature being maintained at about -65°. 950 ml of ethyl acetate, 950 ml of hexane and l.l3 liter of water are added, the temperature of the mixture being about 5°, and the mixture is stirred for 15 minutes, the resulting temperature of the mixture being about 5°. The top organic layer is separated and washed successively with a total of l.4 l of saturated sodium chloride solution (pH 7.5), and the solvent is distilled at 20 to 30 mm Hg at a maximum external temperature of about 45°.
(c) 3.25 liter of ethyl acetate is added to the obtained oil (the predominantly cyclic boronate). 340 ml of 30 % hydrogen peroxide solution (3 moles) is then slowly added so as to maintain an internal temperature of 20° to 25°, and the reaction mixture is stirred at 20° to 25° for about 3 hours until thin layer chromatography shows no boronate present. The top organic layer is washed twice with a total of 1.6 l of saturated sodium chloride solution, pH 7.5. The top organic layer is then separated, washed three times (for ten minutes each time) with a total of l.5 l of 10 % sodium sulfite solution (until the organic layer is free of peroxide) while maintaining an internal temperature of 25°. The top organic layer is washed with 600 ml of saturated sodium chloride solution (pH 7.5). The solvent is distilled at 20 to 30 mm Hg at a maximum external temperature of about 45°. l06 g of crude material is obtained. Purification of 0.68 g of the crude dihydroxy ester is done by column chromatography using ethyl acetate/hexane (l:2) as the eluant, yielding 490 mg (M.P. 143-145°), which is shown by NMR analysis to contain the erythro isomer in 98. 78% purity (there being no threo isomer present); [α]_{D}²⁰ = + 6.49° (c = 0.77, CH₂Cl₂).

### Example 4: (3R,5S)-Erythro-dihydroxy-6-trityloxyhexanoic acid tert-butyl ester

### [Formula Iu: u = trityl; Rᵤ = t-butyl; in (-)-enantiomeric form]

(a) 5.61 g (148.4 mmoles) of sodium borohydride are added to a solvent comprising 990 ml of dry THF and 280 ml of methanol under nitrogen at about -76°. The temperature increases to about -74°. To the resulting solution is added l29.7 ml of 15 % diethylmethoxyborane in THF dropwise over a 20 minute period, and the formed mixture is stirred for an additional l0 minutes at from -77° to -76°.
(b) 56 g (0.122 mmole) of (S)-5-hydroxy-6-trityloxy-3-oxo-hexanoic acid t-butyl ester in l65 ml of THF and 41 ml of methanol at a temperature of from about -77° to -75° are added dropwise to the mixture formed in (a) over a period of 40 minutes, and the resulting mixture is stirred for an additional two hours at from -77° to -75°. l56 ml of saturated ammonium chloride solution is added to quench the reaction. 500 ml of ethyl acetate, 500 ml of heptane and 600 ml of water are then added. The top organic layer is separated and washed successively with a total of 600 ml of saturated sodium chloride solution, pH 7.5, and the organic layer is concentrated at 20 to 30 mm Hg at a maximum external temperature of about 45°.
(c) 793 ml of ethyl acetate is added to the obtained organic residue (containing predominantly the cyclic boronate). 79 ml of 30 % hydrogen peroxide solution (0.7 moles) is then slowly added, and the reaction mixture is stirred for about 3 hours until thin layer chromatography shows no boronate present. The top organic layer is washed twice with a total of 400 ml of saturated sodium chloride solution, pH 7.5. The top organic layer is then separated, washed three times (for ten minutes each time) with a total of 576 ml of 10% sodium sulfite solution (until the organic layer is free of peroxide) while maintaining an internal temperature of 25°. The top organic layer is then washed successively with a total of 200 ml of saturated sodium chloride solution, pH 7.5, and the solvent is distilled at 20-30 mm Hg and a maximum external temperature of about 45°. 54.3 g of the crude dihydroxy compound (M.P. 84-86°) are obtained, which is indicated to contain 99.19 % erythro isomer; [α]_{D}²⁰ = - 5.59° (c = 1.6, CH₂Cl₂).

### 10.2. Examples for the preparation of intermediates of formula VII by process A

### Example 5: 3-(N,N-Dimethylamino)acrolein

### [= (E)-3-(N,N-Dimethylamino)prop-2-enal] [Formula VII: R₁₂, R₁₃ = methyl] [Subprocess Aa]

Step (i): A 12 l four-neck round bottom flask equipped with a stirrer, brine-filled condenser, thermometer, caustic scrubber, addition funnel and cooling bath is charged, under a blanket of nitrogen, with 4.0 l of methylene chloride and 438 g (5.99 moles) of N,N-dimethylformamide. The solution is cooled to 7°, and 860 g (6.8 moles) of oxalyl chloride is added over a period of 2.5 hours at a rate such that little or no solvent and/or reagent is swept into the condenser, while maintaining the temperature of the reaction mixture at 5° to 10°. A white solid forms.
Step (ii): 483 g (6.7 moles) of ethyl vinyl ether is added over a period of 30 to 60 minutes while maintaining a maximum temperature of 25° to 28°, the addition being very exothermic. A brown-red solution results. The reaction mixture is heated at 37° to 38° for 30 minutes, refluxing taking place. As much methylene chloride as possible is recovered by distillation at 30 to 40 mm Hg and 45° and, after the distillation ceases, the reaction mixture is maintained at 30 mm Hg and 45° for 30 minutes to obtain a dark brown stirrable oil.
Step (iii): The reaction mixture is cooled to 20°, and 450 ml of water is added over a period of about 30 minutes; the exotherm is allowed to raise the temperature to 60°, and this temperature is maintained for the balance of the addition. The mixture is stirred at 50° to 60° for 30 minutes and cooled to 20°. A solution of 1.71 kg (12.35 moles) of anhydrous potassium carbonate in 3.6 l of water is added over a period of 30 to 45 minutes while maintaining the temperature at 20° to 22°. The aqueous layer is extracted with 4 l of methylene chloride, the bottom methylene chloride layer is separated, and the top aqueous layer is extracted four times with 1 l portions of methylene chloride. The five methylene chloride phases are combined, dried over 500 g of anhydrous sodium sulfate and filtered, and the solid is washed twice with 250 ml portions of methylene chloride. The washings and filtrate are combined and as much methylene chloride as possible is recovered by distillation at 20 to 40 mm Hg and 45° to obtain a thick stirrable oil.
Step (iv): The oil is cooled to 20°, 500 ml of methanol is added, the mixture is cooled to 10°, and 60 g (1.33 moles) of anhydrous dimethylamine is added while maintaining a maximum temperature of 20°. As much solvent as possible is recovered by distillation at 20 to 30 mm Hg and 70°, the pressure is lowered to 3 to 4mm Hg, and distillation is continued while gradually raising the temperature until it reaches 120° and the vapor temperature reaches 115° to obtain the 89.7 % pure product as an oil [412 g; yield 62 %; B.P. of pure product 271°-272.8°).

### Example 6: 3-(N-methyl-N-phenylamino)acrolein

### [= (E)-3-(N-Methyl-N-phenylamino)prop-2-enal] [Formula VII: R₁₂ = phenyl; R₁₃ = methyl] [Subprocess Ab, variant Ab1]

Step (i): A 12 l four-neck round bottom flask equipped with a stirrer, brine-filled condenser, thermometer, caustic scrubber, addition funnel and cooling bath is charged, under a blanket of nitrogen, with 3.0 l of methylene chloride and 1.02 kg (7.4 moles) of N-methylformanilide. The solution is cooled to 15°, and 1.10 kg (8.67 moles) of oxalyl chloride is added over a period of 1.5 hours at a rate such that little or no solvent and/or reagent is swept into the bottom of the brine-filled condenser, while maintaining a temperature of 15° to 17° under gentle refluxing. The reaction mixture is slowly warmed to 43° over a period of 1 hour, refluxed for 1 hour at 43° to 45° to obtain a clear yellow solution and cooled to 15°.
Step (ii): 648 g (8.99 moles) of ethyl vinyl ether is added over a period of 40 to 60 minutes while maintaining a maximum temperature of 28° to 29°, the reaction being very exothermic. The resulting brown-red solution is heated at 38° to 39° for 30 minutes, refluxing taking place, and is cooled to 15°.
Step (iii): A solution of 960 g (9.05 moles) of anhydrous sodium carbonate in 4.5 l of water is added over a period of 45 to 60 minutes while maintaining a temperature of 22° to 30°, the addition being very exothermic. The mixture is stirred at 22° to 25° for 15 minutes and allowed to stand for 15 minutes to permit separation into two phases. The organic phase is separated, and the aqueous phase is extracted with 1.25 l of methylene chloride. The methylene chloride extract is combined with the previous organic phase, and the combined solution is extracted with 1 l of water. The aqueous extract is back extracted with 250 ml of methylene chloride and this methylene chloride extract is combined with the previous organic phase. As much methylene chloride as possible is recovered by distillation at 20 to 40 mm Hg and 60°, and the residual oil is heated at 20 to 30 mm Hg and 60° to 65° for 4 hours to obtain the 83.5 % pure product as an oil [1.295 kg; yield 90.7 %; B.P. of pure product 244° (dec.); M.P. of pure product 46-47° from isopropanol/hexane 1:1].

### Example 7: 3-(N-Methy-N-phenylamino)acrolein

### [= (E)-3-(N-Methyl-N-phenylamino)prop-2-enal] [Formula VII: R₁₂, R₁₃ = as for Example 6] [Subprocess Ab, variant Ab2]

Step (i): A 5 l four-neck round bottom flask equipped with a stirrer, brine-filled condenser, thermometer, caustic scrubber, addition funnel and cooling bath is charged, under a blanket of nitrogen, with 350 ml of acetonitrile and 425 g (3.8 moles) of N-methylformanilide. The solution is cooled to -15°, and 440 g (3.46 moles) of oxalyl chloride is added over a period of 1.5 hours at a rate such that little or no solvent and/or reagent is swept into the bottom of the brine-filled condenser maintained at -25° to -20°, while maintaining a temperature of -15° to -10° under gentle refluxing. The reaction mixture is slowly warmed to 15° over a period of 30 minutes and stirred for 15 minutes at 15° to 18°.
Step (ii): 339.5 g (3.39 moles) of n-butyl vinyl ether is added over a period of 45 minutes while maintaining a maximum temperature of 28° to 30°, the reaction being very exothermic. The reaction mixture is stirred at 30° to 35° for 30 minutes to obtain a red-brown solution and is cooled to 0°.
Step (iii): A solution of 395 g (3.73 moles) of anhydrous sodium carbonate in 1.75 l of water is added over a period of 40 to 60 minutes while maintaining a temperature of 8° to 10°, the addition being very exothermic. 1.75 l of toluene is added, and the mixture is stirred at 20° to 22° for 15 minutes and allowed to stand for 15 minutes to permit separation into two phases. The organic phase is separated and washed twice with 150 ml portions of water. As much toluene as possible is recovered by distillation at 20 to 80 mm Hg and 60° to 90°, and the residual oil is heated at 20 to 30 mm Hg and 89° to 90° for 30 minutes to obtain the 86.6 % pure product as an oil [492 g; yield 85.7 %; B.P. of pure product 244° (dec.); M.P. of pure product 46-47° from isopropanol/hexane 1:1].

If the reaction mixture is stirred at 28° to 30° for 30 minutes instead of at 30° to 35°, a 90.7 % yield of a 92.3 % pure product is obtained.

### Example 7a: 3-(N-Methyl-N-phenylamino)acrolein

### [= (E)-3-(N-Methyl-N-phenylamino)prop-2-enal] [Formula VII: R₁₂, R₁₃ = as for Example 6] [Subprocess Ab, variant Ab2, neat reagents]

Step (i): A 2.5 l four-neck flask equipped as in Example 7, step (i) is charged, under an atmosphere of nitrogen, with 223.2 ml (1.81 moles) of N-methylformanilide. The solution is cooled to 15° and 177.6 ml (2.07 moles) of oxalyl chloride is added over a period of 20 minutes while maintaining the same temperature. A spontaneous gas evolution occurs and an orange, homogeneous solution forms.
Step (ii): 278.4 ml (2.16 moles) of n-butyl vinyl ether is added over a period of 45 minutes while maintaining an internal temperature of 25° to 30°, the reaction being exothermic. The orange suspension is stirred at 40° to 45° for 30 minutes and is cooled to 0°.
Step (iii): To the product of step (ii) is added slowly during about 90 minutes 4N NaOH solution so that the temperature does not exceed 5°. The mixture is warmed up to room temperature and stirred for a further 60 minutes. The organic layer is separated in a 3 l funnel and the aqueous phase is extracted with 100 ml of n-butanol. The combined organic layers are washed twice with 200 ml of brine and the solvent distilled off at 80°/15 Torr over 2 hours to give a thick, brown-black oil [295 g; yield 92 %; chemical purity >98 %; B.P. of pure product 244° (dec.); M.P. of pure product 46-47° from isopropanol/hexane 1:1].

### Example 8: 3-(N-Methyl-N-phenylamino)acrolein

### [= (E)-3-(N-Methyl-N-phenylamino)prop-2-enal] [Formula VII: R₁₂, R₁₃ = as for Example 6] [Subprocess Ab, variant Ab3]

Steps (i) and (ii): A 12 l four-neck round bottom flask equipped with a stirrer, brine-filled condenser, thermometer, caustic scrubber, addition funnel and cooling bath is charged, under a blanket of nitrogen, with 1.056 kg (8.15 moles) of oxalyl chloride and 480 ml of acetonitrile. The solution is cooled to -10°, and a mixture of 1.02 kg (7.395 moles) of N-methylformanilide, 816 g (7.98 moles) of n-butyl vinyl ether and 360 ml of acetonitrile is added over a period of 2.5 hours at a rate such that little or no solvent and/or reagent is swept into the bottom of the brine-filled condenser (maintained at -25° to -20°), while maintaining a temperature of -10° to -5° under gentle refluxing. The resulting homogeneous orange reaction mixture is slowly warmed to 20° over a period of 30 minutes; a slight exotherm raises the temperature to 28° over a period of 30 minutes. The reaction mixture is stirred at 28° to 30° for 1 hour to obtain a brown homogeneous mixture and is cooled to 0°
Step (iii): A solution of 948 g (8.94 moles) of anhydrous sodium carbonate in 4.20 l of water is added over a period of 45 to 60 minutes while maintaining a temperature of 8° to 10°, the addition initially being very exothermic. 3.60 l of toluene is added, and the mixture is stirred at 20° to 22° for 15 minutes and allowed to stand for 15 minutes to permit separation into two phases. The organic phase is separated and washed twice with 360 ml portions of water. As much toluene as possible is recovered by distillation at 20 to 80 mm Hg and 60° to 90°, and the residual oil is heated at 20 to 30 mm Hg and 89° to 90° for 30 minutes to obtain the 89.1 % pure product as an oil [1.16 kg; yield 86.6 %; B.P. of pure product 244°C. (dec.); M.P. of pure product 46-47° from isopropanol/hexane 1:1].

### 10.3. Examples for the preparation of intermediates of formula Va by process B

### Example 9: (E)-3-[3'-(4''-Fluorophenyl)-1'-(1''-methylethyl)1H-indol-2'-yl]prop-2-enal

### [Formula Va: R₅, R₆ = H; R₇ = 1-methylethyl; R₈ = 4-fluorophenyl] [Process B]

(i) A 5 l four-neck round bottom flask equipped with a stirrer, condenser, thermometer, addition funnel and cooling bath is charged, under a blanket of dry nitrogen, with 100 ml of dry acetonitrile and 174.4 g (1.14 moles) of phosphorus oxychloride, the mixture is cooled to -5°, and a solution of 184 g (0.96 mole) of 83.5 % pure 3-(N-methyl-N-phenylamino)acrolein (product of Examples 6 to 8) in 156 ml of dry acetonitrile is added over a period of 45 minutes while maintaining a temperature of -5° to +5°. The reaction mixture is stirred at 0° to 5° for 10 minutes.
(ii) 115.2 g (0.45 mole) of 3-(4'-fluorophenyl)-1-(1'-methylethyl)-1H-indole(compound of formula XVII) is added over a period of 20 minutes while maintaining a temperature of about 5°. The reaction mixture is refluxed at 83° for 9 hours and cooled to 10°.
(iii) A solution of 228 g (5.7 moles) of sodium hydroxide in 2.0 l of water is slowly added over a period of 30 minutes while maintaining a temperature of 25° to 30°, the addition being very exothermic. 1.6 l of toluene is added, the mixture is stirred at 25° for 30 minutes and filtered through a filter pad. The filter cake is washed with 100 ml of toluene, and the washing is combined with the previous filtrate. The organic layer is separated, and a mixture of 93.4 g of concentrated hydrochloric acid and 2 l of water is added followed by 400 ml of saturated sodium chloride solution. The mixture is stirred at 25° for 30 minutes, and the resulting slurry is filtered through a filter pad. The tars are washed with 100 ml of toluene, and the washing is combined with the filtrate. The organic layer is separated, washed twice with 2 l portions of deionized water and filtered through a filter pad. As much toluene as possible is recovered by distillation at 30-50 mm Hg and an external temperature of 60° to 65° to obtain a thick stirrable oil. 100 ml of 95 % ethanol is added, as much ethanol as possible is recovered by distillation at 30 to 80 mm Hg and 60° to 65°, and this is repeated twice. 180 ml of 95 % ethanol is added, and the mixture is refluxed at 78° for 15 minutes and slowly cooled to 20° over a period of 2 hours, crystallization commencing at about 55°. The slurry is slowly cooled to 0° to 5° over a period of 30 minutes, maintained at 0° to 2° for 1 hour and filtered. The filter cake is washed three times with 50 ml portions of cold (0° to 5°) 95 % ethanol and vacuum dried at 60° to 65° for 16 hours to constant weight to obtain the 98.7 % pure product (101 g; yield 71.3 %; M.P. 127°-128°).

In a variant isopropanol is used in place of 95 % ethanol.

### Example 10: (E)-3-[3'-(4''-Fluorophenyl)-1'-(1''-methylethyl)-1H-indol-2'-yl]prop-2-enal

### [Formula Va: R₅, R₆, R₇, R₈ = as for Example 9] [Process B, alternative procedure]

i) A 5 l four-neck round bottom flask equipped with a stirrer, condenser, thermometer, addition funnel and cooling bath is charged, under a blanket of dry nitrogen, with 263 ml of dry acetonitrile and 454 g (2.96 moles) of phosphorus oxychloride, the mixture is cooled to -5°, and a solution of 471.6 g (2.49 moles) of 85.5 % pure 3-(N-methyl-N-phenylamino)acrolein in 406 ml of dry acetonitrile is added over a period of 45 minutes while maintaining a temperature of 5° to 7°. The reaction mixture is stirred at 5° to 7° for 10 minutes.
ii) 300 g (1.18 moles) of 3-(4'-fluorophenyl)1-(1'-methylethyl)-1H-indole (compound of formula XVII) is added over a period of 10 minutes while maintaining a temperature of about 7°. The reaction mixture is refluxed at 83° for 3 hours and cooled to 22°.
iii) 2.7 l of water is slowly added over a period of 15 minutes while maintaining a temperature of 22° to 35°, the addition being exothermic. The reaction mixture is stirred at 35° to 50° for 30 minutes, heated at 50° to 55° for 1.5 hours (a longer period of heating may be necessary for complete hydrolysis), cooled to 22°, maintained at 22° for 15 minutes and filtered. The filter cake is washed three times with 600 ml portions of water and suction dried at aspirator pressure for 6 to 16 hours (N-methylaniline may be recovered from the combined aqueous layer and washings). The wet filter cake is transferred to the original 5 l flask, 2.5 l of toluene and 180 g of 20 µ powdered cellulose are added, and the mixture is heated at 50° to 55° for 1.5 hours, cooled to 22°, maintained at 22° for 15 minutes and optionally filtered through a pad of 91 g of 70-230 mesh A.S.T.M. silica gel covered with a filter cloth. The cellulose and silica gel pad are washed three times with 200 ml portions of toluene. The toluene filtrate and washings are combined and as much toluene as possible is recovered by distillation at 30 to 50 mm Hg and 50° to 65° (external). 280 ml of 95 % ethanol is added to the residual thick oil, the ethanol is distilled at 20 to 30 mm Hg and 60° to 65°, 280 ml of 95 % ethanol is added, and as much ethanol as possible is distilled at 30 to 80 mm Hg and 60° to 65°. 700 ml of 95 % ethanol is added, and the mixture is refluxed at 78° for 15 minutes and slowly cooled to 20° over a period of 1 hour, crystallization commencing at about 55°. The resulting slurry is cooled to 0° to 5° over a period of 30 minutes and maintained at 0° to 2° for 30 minutes, and the solids are collected by filtration, washed three times with 120 ml portions of cold (0° to 5°) 95 % ethanol and vacuum dried at 60° to 65° for 16 hours to constant weight to obtain the 99.4 % pure product (276.6 g; yield 75.5 %; M.P. 129°-130°).

In a variant isopropanol is used in place of 95 % ethanol.

It is preferred to omit the pad of silica gel, i.e. the powdered cellulose-containing liquid is subjected to a simple filtration, and it is the residue therefrom that is washed three times with 200 ml portions of toluene.

### Example 10a: (E)-3-[3'-(4''-Fluorophenyl)-1'-(1''-methylethyl)-1H-indol-2'-yl]prop-2-enal

### [Formula Va: R₅, R₆, R₇, R₈ = as for Example 9] [Process B, alternative procedure]

(i) A 1.5 l flask equipped as described in Example 10, step (i) is charged, under a blanket of dry nitrogen, with 170 ml of dry acetonitrile and 105.3 g 3-(N-methyl-N-phenylamino)acrolein hydrochloride salt at room temperature. To the mixture is added over 5 minutes 96.6 g of phosphorus oxychloride. A dark solution is obtained.
(ii) 90 g of 3-(4'-fluorophenyl)-1-(1'-methylethyl)-1H-indole (compound of formula XVII) Is added at 30°. The mixture is heated to reflux for 4.5 hours at 75° to 83°, then cooled to 22°.
(iii) 250 ml of water at 5° are added, followed by 500 ml of water at room temperature over 15 minutes. The mixture is stirred at 35° to 50° for 30 minutes, then heated at 50° to 55° for 1.5 hour. A dark slurry is obtained. The mixture is cooled to 30°, maintained at 30° for 15 minutes, and the brown slurry is filtered. The filter cake is washed three times with a total of 540 ml of water. The filter cake is suction dried under vacuum for about 4 hours. The solids are transferred into the original 1.5 l flask, 750 ml of toluene and 54 g of 20 µ powdered cellulose are added, and the subsequent working-up is effected as described in Example 10, step (iii) to obtain the product (89 g; yield 81 %; M.P. 123-129°).

### 10.4. Examples for the specific embodiment

### Example 11: (+)Erythro-(E)-3,5-dihydroxy-7-[3'-(4''-fluorophenyl)-1'-(1''-methylethyl)indol-2'-yl]hept-6-enoic acid sodium salt

### [Formula Ia: in racemic form; sodium salt form]

Steps (a), (b) and (c): N-Methylformanilide is reacted with oxalyl chloride and ethyl or n-butyl vinyl ether according to process A, subprocess Ab to produce 3-(N-methyl-N-phenylamino)acrolein as described in Examples 6, 7, 7a or 8 [steps (i), (ii) and (iii)].
Step (d): The above product, 3-(N-methyl-N-phenylamino)acrolein, is reacted with phosphorus oxychloride as described in Example 9, 10 or 10a, step (i) to produce the compound of formula XVI wherein Xₐ is chloro, R_{12b} is phenyl and R₁₃ is methyl.
Step (e): The above compound of formula XVI is reacted with 3-(4'-fluorophenyl)-1'-(1'-methylethyl)-1H-indole as described in Example 9, 10 or 10a, step (ii) to produce the compound of formula XVIIIa wherein Xₐ is chloro, R_{12b} is phenyl and R₁₃ is methyl.
Step (f): The above compound of formula XVIIIa is hydrolyzed as described in Example 9, 10 or 10a, Step (iii) to produce (E)-3-[3'-(4''-fluorophenyl)-1'-(1''-methylethyl)-1H-indol-2'-yl]prop-2-enal.
Step (g): Under a nitrogen atmosphere a reactor is charged with 0.5 l of tetrahydrofuran, the solution is cooled to -10°, 60 g sodium hydride (60 % dispersion in mineral oil) are added carefully. Then 237.3 g t-butyl acetoacetate in 250 ml of THF are added carefully over 45 minutes while maintaining the temperature below 2°. The resultant solution is stirred at -10° to 20° for 1 hour. The mixture is cooled to -10° and 938 ml of a 1.6 M solution of n-butyllithium in hexane is added at a rate such that the temperature does not exceed 0° (over about 60 minutes). The mixture is stirred for 10 minutes at that temperature, then cooled to -10°, and a solution of 230 g of product of step f) above in 650 ml of THF is added at a rate such that the temperature does not exceed 0° (over about 70 minutes). The reaction mixture is stirred at 0° for 15 minutes and poured onto a mixture of 248 ml of conc. hydrochloric acid and 2.5 kg of ice under vigourous stirring over 5 to 10 minutes. The mixture is vigourously stirred for a further 15 minutes, the organic phase is separated, washed twice with 500 ml portions of saturated NaCl solution and concentrated under reduced pressure (about 25 mm Hg). To the residue is added 200 ml of toluene and the solution is concentrated again. The obtained crude (+)-(E)-7-[3'-(4''-fluorophenyl)-1'-(1''-methylethyl)indol-2'-yl]-5-hydroxy-3-oxohept-6-enoic acid t-butyl ester (compound of formula IIa wherein R₁ is t-butyl, in racemic form) (503.6 g; 70.04 % pure) is used in the next step without further purification.
Step (h): The above crude product is stereoselectively reduced as described in Example 1, steps (a), (b) and (c) to produce (+)-erythro-(E)-7-[3'-(4''-fluorophenyl)-1'-(1''-methylethyl)idol-2'-yl]-3,5-dihydroxy-hept-6-enoic acid t-butyl ester.
Step (i): To 42.5 g of the ester obtained under step (h) above in 275 ml of THF is added over 5 minutes 90 ml of 1 N sodium hydroxide while maintaining the temperature below 10°. The solution is stirred for 1 hour at room temperature, 275 ml of methanol are added, the mixture is concentrated at 25 mm Hg and 45°, then 300 ml of deionized water are added, distillation is continued to a remaining volume of 140 ml, then 380 ml of deionized water are added again and the solution is washed with a total of 640 ml of t-butyl methyl ether in 3 portions. The aqueous layer is concentrated at 25 mm Hg and 45° to a volume of about 300 ml, 220 ml of deionized water are added, and the clear aqueous solution is lyophilized over 3 days. The title compound is obtained (35.9 g; 91 % yield; chemical purity 98.9 %; 99.9 % pure erythro isomer; boron concentration below detection limit).
   Alternative procedure for step (i): To 35.0'g of the ester obtained under step (h) above in 175 ml of ethanol is added over 5 minutes under stirring 74 ml of 1 N sodium hydroxide solution while maintaining the temperature below 12°. The solution is stirred for 1 hour, the mixture is concentrated at 25 mm Hg and 45°, then 250 ml of deionized water are added, distillation is continued to a remaining volume of 115 ml, then 315 ml of deionized water are added and the solution is washed with a total of 525 ml of tert-butyl methyl ether in 3 portions. The aqueous layer is concentrated at 25 mm Hg and 45° to a volume of about 245 ml, 185 ml of deionized water are added, and the clear aqueous solution is lyophylized over 3 days. The title compound is obtained (29.75 g; colour pure white; 91 % yield; M.P. 204-207° (dec.); chemical purity 100 %; 99.61 % pure erythro isomer; boron concentration 3.96 ppm).

### Example 12: (+)-Erytho-(E)-3,5-dihydroxy-7-[3'-(4''-fluorophlenyl-1'-(1''-methylethyl)indol-2'-yl]hept-6-enoic acid sodium salt

### [Formula Ia: in racemic form; sodium salt form]

The title compound is obtained in a manner analogous to Example 11, except that
- in step (g) the methyl ester is produced, by reaction with the dianion of methyl acetoacetate instead of with t-butyl acetoacetate;
- step (h) is effected as described in Example 2, steps (a), (b) and (c);
- step (i) is effected by hydrolyzing the methyl ester obtained in step (h), as described in USP 4 739 073, Example 6(b) on column 50.

## Claims

1. A process for the preparation of a compound of formula I 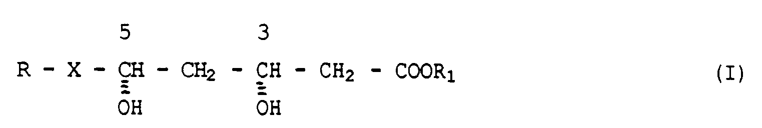 wherein
X is
-CH₂CH₂- or -CH=CH-;
R₁ is an ester group inert to the reaction conditions; and
R is an organic radical having groups which are inert under reducing conditions,
with the proviso that, when R is triphenylmethyl, then R₁ additionally includes allyl and X is -OCH₂-, by stereoselective reduction of a racemic or optically pure compound of formula II 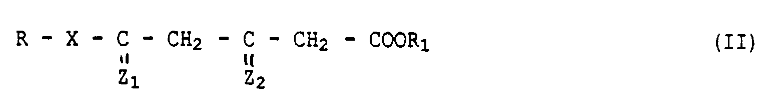 wherein
R, R₁ and X are as defined above and
one of Z₁ and Z₂ is oxygen and the other is hydroxy and hydrogen,
comprising
according to a **first step [step (a)]**, mixing a compound of formula III
R₄O-B-(R₃)₂ (III)
wherein
R₄ is allyl or lower alkyl having from 1 to 4 carbon atoms and
R₃ is a primary or secondary alkyl having 2 to 4 carbon atoms,
with sodium borohydride NaBH₄ in a reaction medium comprising an
alcohol and tetrahydrofuran, in a **second step [step (b)]**, treating a compound of formula II with the mixture obtained in step (a) under conditions suitable to obtain a mixture containing a cyclic boronate compound of formula IV(a) 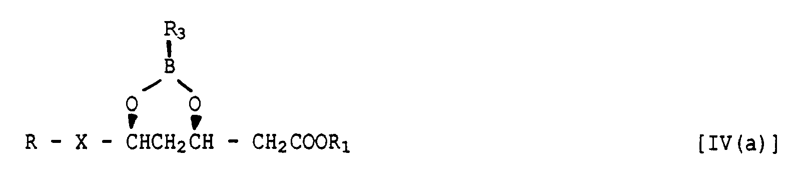 and/or a boron complex of formula IV(b) 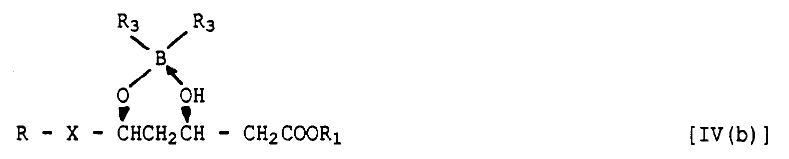 wherein R, R₁, R₃ and X are as defined above, and
in a **third step [step (c)]**, cleaving the product obtained in step (b) to obtain a corresponding compound of formula I,
and if desired, converting the obtained compound of formula I by conventional means into the free acid form, a salt form, a further ester form or the δ-lactone, i e. internal ester, form.

2. A process according to claim 1 for preparing a compound of formula Iu 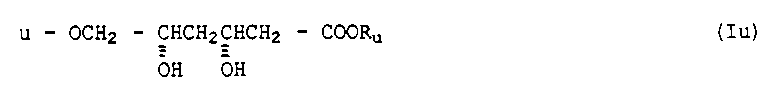 wherein
u is triphenylmethyl (trityl) and
Rᵤ is allyl or a radical forming an ester inert under the reaction conditions, preferably allyl or C₁₋₃alkyl, n-butyl, i-butyl, t-butyl or benzyl, especially t-butyl,
by stereoselectively reducing a racemic or optically pure compound of formula IIu 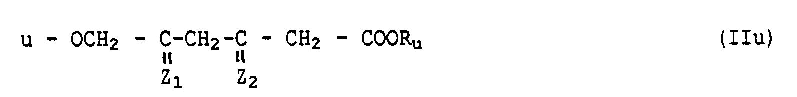 wherein u, Rᵤ, Z₁ and Z₂ are as defined in this claim.

3. A process according to claim 1 for preparing the compound of formula Ia 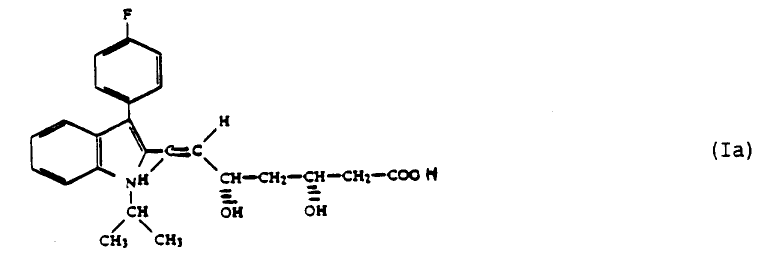 in racemic or optically pure form; in free acid, salt, ester or δ-lactone, i.e. internal ester, form,
and including, if desired, converting the obtained compound of formula Ia in ester form by conventional means into the free acid form, a salt form, a further ester form or the δ-lactone, i.e. internal ester, form.

4. A process according to claim 1 in which a precursor of formula Va 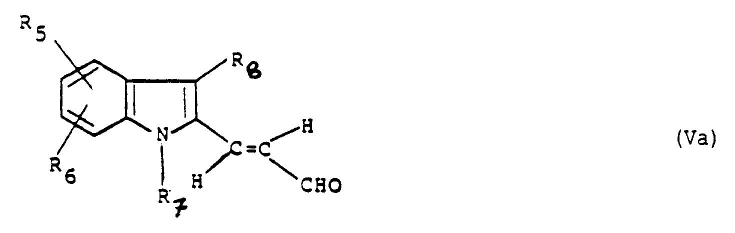 wherein
R₅ is hydrogen, C₁₋₃alkyl, n-butyl, i-butyl, t-butyl, C₃₋₆cycloalkyl, C₁₋₃alkoxy, n-butoxy, i-butoxy, trifluoromethyl, fluoro, chloro, phenoxy or benzyloxy;
R₆ is hydrogen, C₁₋₃alkyl, C₁₋₃alkoxy, trifluoromethyl, fluoro, chloro, phenoxy or benzyloxy;
with the provisos that not more than one of R₅ and R₆ is trifluoromethyl, not more than one of R₅ and R₆ is phenoxy, and not more than one of R₅ and R₆ is benzyloxy;
one of R₇ and R₈ is phenyl trisubstituted by R₉, R₁₀ and R₁₁ and the other is primary or secondary C₁₋₆alkyl not containing an asymmetric carbon atom, C₃₋₆cycloalkyl or phenyl-(CH₂)ₘ-, wherein
R₉ is hydrogen, C₁₋₃alkyl, n-butyl, i-butyl, t-butyl, C₁₋₃alkoxy, n-butoxy, i-butoxy, trifluoromethyl, fluoro, chloro, phenoxy or benzyloxy;
R₁₀ is hydrogen, C₁₋₃alkyl, C₁₋₃alkoxy, trifluoromethyl, fluoro, chloro, phenoxy or benzyloxy;
R₁₁ is hydrogen, C₁₋₂alkyl, C₁₋₂alkoxy, fluoro or chloro, and
m is 1, 2 or 3;
with the provisos that not more than one of R₉ and R₁₀ is trifluoromethyl, not more than one of R₉ and R₁₀ is phenoxy, and not more than one of R₉ and R₁₀ is benzyloxy;
for the starting material of formula II is prepared as follows:
(i) reacting a compound of formula Villa
OHC - N(R_{12b})R₁₃ (VIIIa)
wherein
R_{12b} is phenyl or phenyl substituted by 1 to 3 substituents each of which is independently C₁₋₃alkyl, C₁₋₃alkoxy, fluoro, chloro, bromo or nitro with a maximum of two nitro groups; and
R₁₃ independently has the significance indicated above for R_{12b} or is C₁₋₃alkyl,
with a compound of formula IX
Xₐ - CO-CO - Xₐ (IX)
wherein Xₐ is a monovalent leaving group,
optionally in an inert anhydrous organic medium, to form the corresponding compound of formula Xc
Xₐ- CH=N⁺(R_{12b})R₁₃ Xₐ⁻ (Xc)
wherein Xₐ, R_{12b} and R₁₃ are as defined in this claim,
(ii) reacting that compound of formula X
with a compound of formula XI
R₁₄O - CH=CH₂ (XI)
wherein R₁₄ is a monovalent group that does not deactivate the oxygen atom to which it is attached, optionally in an inert anhydrous organic medium,
to form a corresponding compound of formula XIIc
(E) - R₁₄O - CH=CH - CH=N⁺(R_{12b})R₁₃ Xₐ⁻ (XIIc)
wherein R_{12b}, R₁₃, R₁₄ and Xₐ are as defined in this claim, and
(iii) hydrolyzing that compound of formula XIIc
to obtain a corresponding compound of formula VIIc
(E) - OHC - CH=CH - N(R_{12b})R₁₃ (VIIc)
wherein R_{12b} and R₁₃ are as defined in this claim, in free base or acid addition salt form and, if in acid addition salt form,
neutralizing the acid addition salt with base,
(iv) reacting that compound of formula VIIc
with a compound of formula XV
PO(X_{b})₃ (XV)
wherein X_{b} is chloro or bromo, or
with a compound selected from oxalyl chloride or bromide;
phosgene or carbonyl bromide; phosphorus trichloride or tribromide; phosphorus pentachloride or pentabromide; and an alkyl- or arylsulfonyl chloride or bromide, such as p-toluenesulfonyl chloride or bromide or methanesulfonyl chloride or bromide;
to form the corresponding compound of formula XVI
(E) - X_{b} - CH=CH-CH=N⁺(R_{12b})R₁₃ (XVI)
and the corresponding anion, e.g. ⁻PO₂(X_{b})₂,
wherein X_{b}, R_{12b} and R₁₃ are as defined in this claim,
(v) reacting that compound of formula XVI
with a compound of formula XVII 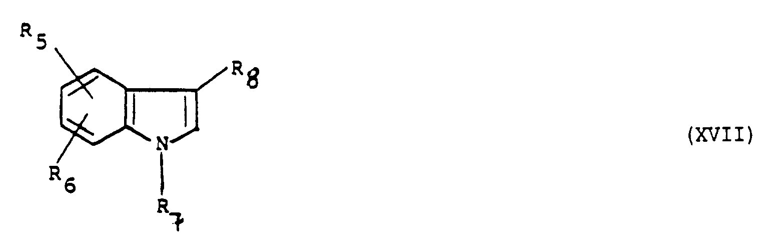 wherein R₅, R₆, R₇ and R₈ are as defined in this claim,
to form a corresponding compound of formula XVIII 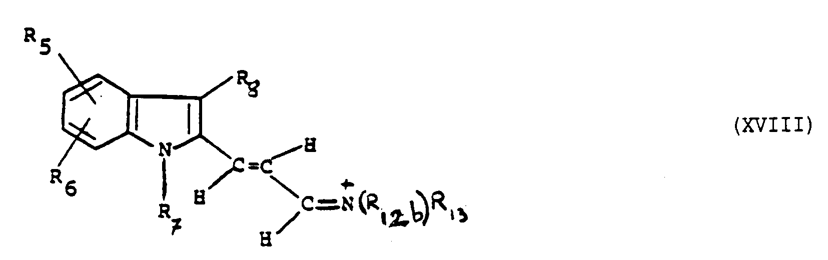 and the corresponding anion, e.g. ⁻PO₂(X_{b})₂,
wherein R₅, R₆, R₇, R₈, R_{12b}, R₁₃ and X_{b} are as defined in this claim and
(vi) hydrolyzing that compound of formula XVIII to obtain a corresponding compound of formula Va.

5. A process according to claim 4 wherein step (iii) is omitted and the compound of formula XII is used directly in place of a compound of formula VIIc for further processing.

6. A process according to claim 4 wherein steps (i) and (ii) are carried out simultaneously.

7. A process according to claim 4 wherein in steps (i) and/or (ii) the reagents are used neat.

8. A process according to claim 4 wherein the compound of formula XII is in acid addition salt form.

9. A process according to claim 3 in which the starting material of formula II is of formula IIa 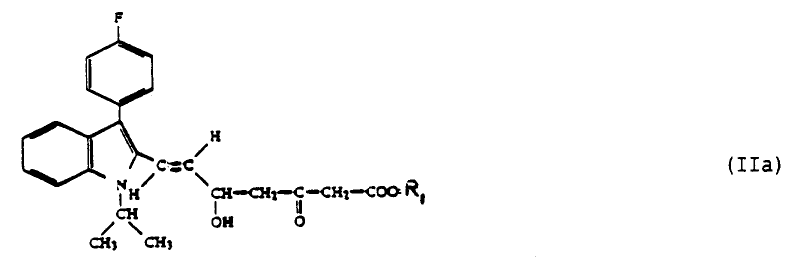 wherein R₁ is as defined in claim 1; in racemic or optically pure form;
and is prepared as follows:
a) reacting a compound of formula VIIIa as defined in claim 4 with a compound of formula IX as defined in claim 4, optionally in an inert anhydrous organic medium, to form a corresponding compound of formula Xc as defined in claim 4,
b) reacting that compound of formula Xc with a compound of formula XI as defined in claim 4, optionally in an inert anhydrous organic medium, to form a corresponding compound of formula XIIc as defined in claim 4,
c) hydrolyzing that compound of formula XIIc to obtain a corresponding compound of formula VIIc as defined in claim 4 in free base or acid addition salt form and, if in acid addition salt form, neutralizing the acid addition salt with base;
d) reacting that compound of formula VIIc
with a compound of formula XV as defined in claim 4 or with a compound selected from oxalyl chloride or bromide;
phosgene or carbonyl bromide; phosphorus trichloride or tribromide; phosphorus pentachloride or pentabromide; and an alkyl- or arylsulfonyl chloride or bromide, such as p-toluenesulfonyl chloride or bromide or methanesulfonyl chloride or bromide;
to form a corresponding compound of formula XVI as defined in claim 4 and the corresponding anion, e.g. ⁻PO₂(X_{b})₂;
e) reacting that compound of formula XVI with
3-(4'-fluorophenyl)-1-(1'-methylethyl)-1H-indole (the compound of formula XVII wherein R₅ and R₆ are hydrogen, R₇ is 1-methylethyl and R₈ is p-fluorophenyl)
to form a corresponding compound of formula XVIIIa 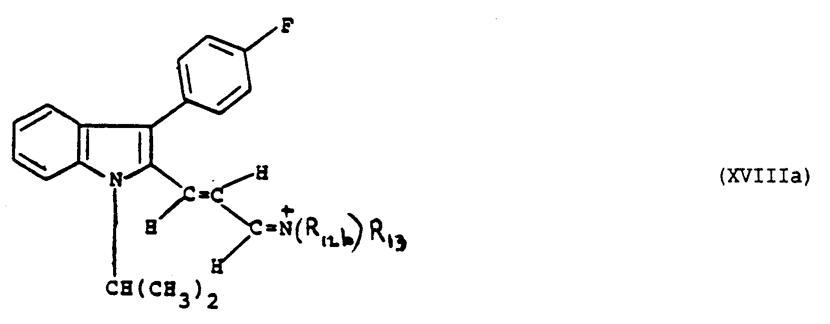 and the corresponding anion, e.g. ⁻PO₂(X_{b})₂,
wherein R_{12b}, R₁₃ and X₆ are as defined in claim 4;
f) hydrolyzing that compound of formula XVIIIa to obtain (E)-3-[3'-(4''-fluorophenyl)-1'-(1''-methylethyl)-1H-indol-2'-yl]-prop-2-enal (the compound of formula Va wherein R₅ and R₆ are hydrogen, R₇ is 1-methylethyl and R₈ is p-fluorophenyl); and
g) reacting that compound of formula Va with the dianion of an acetoacetic ester of formula CH₃COCH₂COOR₁ wherein R₁ is as defined in claim 1.

10. A process according to claim 3 wherein the compound of formula Ia is obtained in racemic form.

11. A process according to claim 3 wherein the compound of formula Ia is obtained in (3R,5S) enantiomeric form.

12. A process according to claim 3 wherein the compound of formula Ia is obtained in sodium salt form.

13. A process according to claim 4 wherein R_{12b} is phenyl and R₁₃ is methyl.

14. A process according to claim 3 wherein R₁ is t-butyl.

## Patentansprüche

1. Verfahren zur Herstellung einer Verbindung der Formel I: 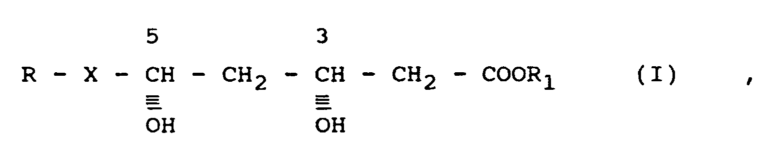 worin
X -CH₂CH₂- oder -CH=CH- ist;
R₁ eine bei den Reaktionsbedingungen inerte Estergruppe ist; und
R ein organischer Rest ist mit Gruppen, die unter reduzierenden Bedingungen inert sind;
mit dem Vorbehalt, daß dann, wenn R ein Triphenylmethylrest ist, R₁ zusätzlich einen Allylrest einschließt und X -OCH₂- ist, durch stereoselektive Reduktion einer razemischen oder optisch reinen Verbindung der Formel II: 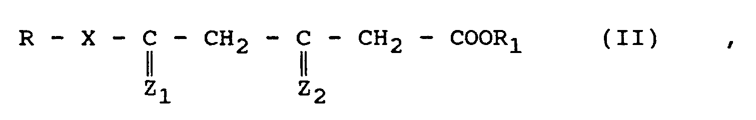 worin R, R₁ und X wie oben definiert sind und einer der Reste Z₁ und Z₂ Sauerstoff ist und der andere ein Hydroxyrest oder Wasserstoff ist,
umfassend, daß man
in einer ersten Stufe [Stufe (a)] eine Verbindung der Formel III:
R₄O-B-(R₃)₂ (III),
worin
R₄ ein Allylrest oder Niedrigalkylrest mit 1 bis 4 Kohlenstoffatomen ist und
R₃ ein primärer oder sekundärer Alkylrest mit 2 bis 4 Kohlenstoffatomen ist,
mit Natriumborhydrid NaBH₄ in einem Reaktionsmedium, das Alkohol und Tetrahydrofuran umfaßt, vermischt,
in einer zweiten Stufe [Stufe (b)] eine Verbindung der Formel 11 mit der in Stufe (a) erhaltenen Mischung unter Bedingungen behandelt, die geeignet sind, um eine Mischung zu erhalten, die eine cyclische Boronatverbindung der Formel IV(a): 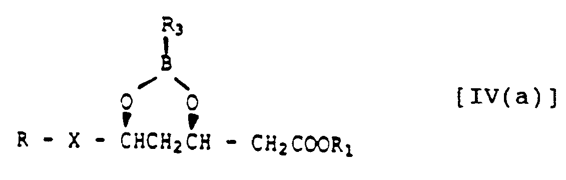 und/oder einen Borkomplex der Formel IV(b): 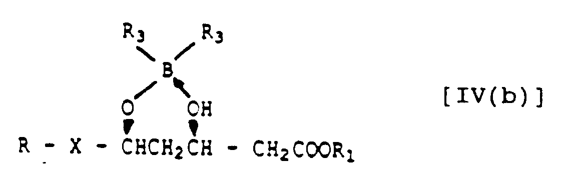 enthält, worin R, R₁, R₃ und X wie oben definiert sind, und in einer dritten Stufe [Stufe (c)] das in Stufe (b) erhaltene Produkt spaltet, um eine entsprechende Verbindung der Formel I zu erhalten, und, falls erwünscht, die erhaltene Verbindung der Formel I mit üblichen Mitteln in die Form der freien Säure, eines Salzes, eines weiteren Esters oder des δ-Lactons, d.h. eines inneren Esters, umwandelt.

2. Verfahren nach Anspruch 1 zur Herstellung einer Verbindung der Formel Iu: 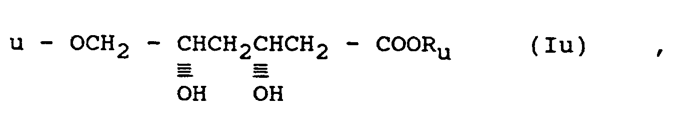 worin
u ein Triphenylmethyl(Trityl)rest ist und
Rᵤ ein Allylrest oder ein Rest ist, der unter den Reaktionsbedingungen einen inerten Ester bildet, Vorzugsweise ein Allylrest oder ein C₁-C₃-Alkyl-, n-Butyl-, i-Butyl-, t-Butyl- oder Benzylrest ist, insbesondere ein t-Butylrest ist,
durch stereoselektive Reduktion einer razemischen oder optisch reinen Verbindung der Formel IIu: 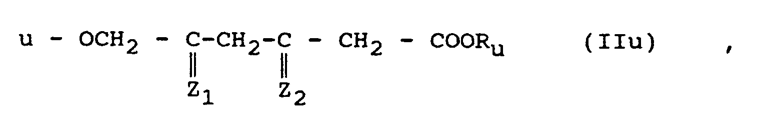 worin u, Rᵤ, Z₁ und Z₂ wie in diesem Anspruch definiert sind.

3. Verfahren nach Anspruch 1 zur Herstellung der Verbindung der Formel Ia: 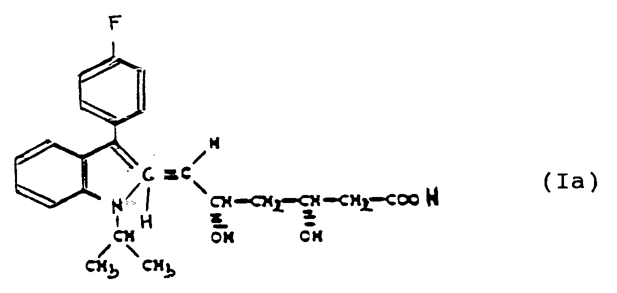 in razemischer oder optisch reiner Form, in Form der freien Säure, des Salzes, des Esters oder des δ-Lactons, d.h. des inneren Esters, und einschließend, falls erwünscht, daß die erhaltene Verbindung der Formel Ia in Esterform mit üblichen Mitteln in die freie Säure, das Salz, einen weiteren Ester oder das δ-Lacton, d.h. den inneren Ester, umgewandelt wird.

4. Verfahren nach Anspruch 1, worin ein Vorläufer der Formel Va: 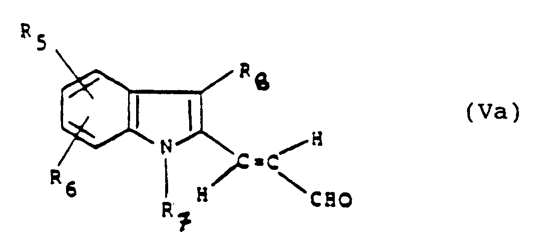 worin
R₅ Wasserstoff, ein C₁-C₃-Alkyl-, n-Butyl-, i-Butyl-, t-Butyl-, C₃-C₆-Cycloalkyl-, C₁-C₃-Alkoxy-, n-Butoxy-, i-Butoxy-, Trifluormethyl-, Fluor-, Chlor-, Phenoxy- oder Benzyloxyrest ist;
R₆ Wasserstoff, ein C₁-C₃-Alkyl-, C₁-C₃-Alkoxy-, Trifluormethyl-, Fluor-, Chlor-, Phenoxy- oder Benzyloxyrest ist;
mit den Vorbehalten, daß nicht mehr als einer der Reste R₅ und R₆ ein Trifluormethylrest ist, nicht mehr als einer der Reste R₅ und R₆ ein Phenoxyrest ist und nicht mehr als einer der Reste R₅ und R₆ ein Benzyloxyrest ist;
einer der Reste R₇ und R₈ ein mit R₉, R₁₀ und R₁₁ trisubstituierter Phenylrest ist und der andere ein primärer oder sekundärer C₁-C₆-Alkylrest ist, der kein asymmetrisches Kohlenstoffatom enthält, ein C₃-C₆-Cycloalkyl- oder Phenyl-(CH₂)ₘ-Rest ist, worin
R₉ Wasserstoff, ein C₁-C₃-Alkyl-, n-Butyl-, i-Butyl-, t-Butyl-, C₁-C₃-Alkoxy-, n-Butoxy-, i-Butoxy-, Trifluormethyl-, Fluor-, Chlor-, Phenoxy- oder Benzyloxyrest ist;
R₁₀ Wasserstoff, ein C₁-C₃-Alkyl-, C₁-C₃-Alkoxy-, Trifluormethyl-, Fluor-, Chlor-, Phenoxy- oder Benzyloxyrest ist;
R₁₁ Wasserstoff, ein C₁-C₂-Alkyl-, C₁-C₂-Alkoxy-, Fluor- oder Chlorrest ist und
m 1, 2 oder 3 ist;
mit den Vorbehalten, daß nicht mehr als einer der Reste R₉ und R₁₀ ein Trifluormethylrest ist, nicht mehr als einer der Reste R₉ und R₁₀ ein Phenoxyrest ist und nicht mehr als einer der Reste R₉ und R₁₀ ein Benzyloxyrest ist;
für das Ausgangsmaterial von Formel II wie folgt hergestellt wird:
(i) daß man eine Verbindung der Formel VIIIa:
OHC - N(R_{12b})R₁₃ (VIIIa) ,
worin
R_{12b} ein Phenylrest oder ein mit 1 bis 3 Substituenten substituierter Phenylrest ist, wobei jeder der Substitutenten unabhängig ein C₁-C₃-Alkyl-, C₁-C₃-Alkoxy-, Fluor-, Chlor-, Brom- oder Nitrorest ist, bei einem Maximum von zwei Nitrogruppen; und
R₁₃ unabhängig die Bedeutung hat, die oben für R_{12b} angegeben ist, oder ein C₁-C₃-Alkylrest ist,
mit einer Verbindung der Formel IX:
Xₐ - CO-CO - Xₐ (IX) ,
worin Xₐ eine monovalente Abgangsgruppe ist,
gegebenenfalls in einem inerten wasserfreien organischen Medium umsetzt unter Bildung der entsprechenden Verbindung der Formel Xc:
Xₐ - CH=N⁺(R_{12b})R₁₃ Xₐ⁻ (Xc) ,
worin Xₐ, R_{12b} und R₁₃ wie in diesem Anspruch definiert sind,
(ii) diese Verbindung der Formel X mit einer Verbindung der Formel XI:
R₁₄O - CH=CH₂ (XI) ,
worin R₁₄ eine monovalente Gruppe ist, die das Sauerstoffatom, an das sie gebunden ist, nicht desaktiviert,
gegebenenfalls in einem inerten wasserfreien organischen Medium umsetzt unter Bildung einer entsprechenden Verbindung der Formel XIIc:
(E) - R₁₄O - CH=CH - CH=N⁺(R_{12b})R₁₃ Xₐ⁻ (XIIc) ,
worin R_{12b}, R₁₃, R₁₄ und Xₐ wie in diesem Anspruch definiert sind, und
(iii) diese Verbindung der Formel XIIc hydrolysiert, um eine entsprechende Verbindung der Formel VIIc:
(E) - OHC - CH=CH - N(R_{12b})R₁₃ (VIIc) ,
worin R_{12b} und R₁₃ wie in diesem Anspruch definiert sind,
in Form der freien Base oder des Säureadditionssalzes zu erhalten, und, wenn die Verbindung in Form des Säureadditionssalzes ist, das Säureadditionssalz mit Base neutralisiert,
(iv) diese Verbindung der Formel VIIc mit einer Verbindung der Formel XV:
PO(X_{b})₃ (XV) ,
worin X_{b} Chlor oder Brom ist,
oder mit einer Verbindung ausgewählt aus Oxalylchlorid oder Oxalylbromid; Phosgen oder Carbonylbromid; Phosphortrichlorid oder -tribromid; Phosphorpentachlorid oder -pentabromid; und einem Alkyl- oder Arylsulfonylchlorid oder -bromid, zum Beispiel p-Toluolsulfonylchlorid oder -bromid oder Methansulfonylchlorid oder -bromid, umsetzt unter Bildung der entsprechenden Verbindung der Formel XVI:
(E) - X_{b} - CH=CH-CH=N+⁺(R_{12b})R₁₃ (XVI)
und des entsprechenden Anions, zum Beispiel ⁻PO₂(X_{b})₂,
worin X_{b}, R_{12b} und R₁₃ wie in diesem Anspruch definiert sind,
(v) diese Verbindung der Formel XVI mit einer Verbindung der Formel XVII: 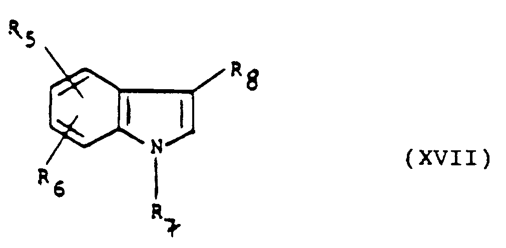 worin R₅, R₆, R₇ und R₈ wie in diesem Anspruch definiert sind, umsetzt unter Bildung einer entsprechenden Verbindung der Formel XVIII: 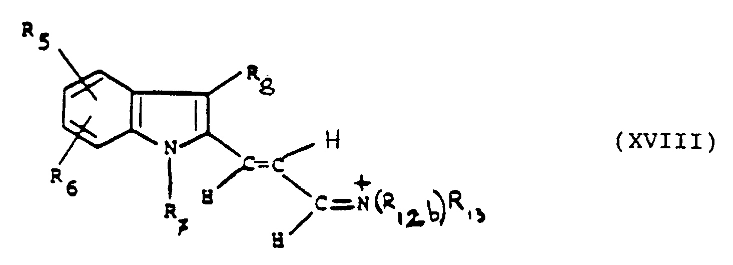 und des entsprechenden Anions, zum Beispiel ⁻PO₂(X_{b})₂, worin R₅, R₆, R₇, R₈, R_{12b}, R₁₃ und X_{b} wie in diesem Anspruch definiert sind, und
(vi) diese Verbindung der Formel XVIII hydrolysiert, um die entsprechende Verbindung der Formel Va zu erhalten.

5. Verfahren nach Anspruch 4, worin Stufe (iii) weggelassen wird und die Verbindung der Formel XII direkt anstelle einer Verbindung der Formel VIIc für die weitere Aufarbeitung verwendet wird.

6. Verfahren nach Anspruch 4, worin die Stufen (i) und (ii) gleichzeitig durchgeführt werden.

7. Verfahren nach Anspruch 4, worin in den Stufen (i) und/oder (ii) die Reagenzien rein verwendet werden.

8. Verfahren nach Anspruch 4, worin die Verbindung der Formel XII in Form des Säureadditionssalzes ist.

9. Verfahren nach Anspruch 3, worin das Ausgangsmaterial der Formel II die Formel IIa: 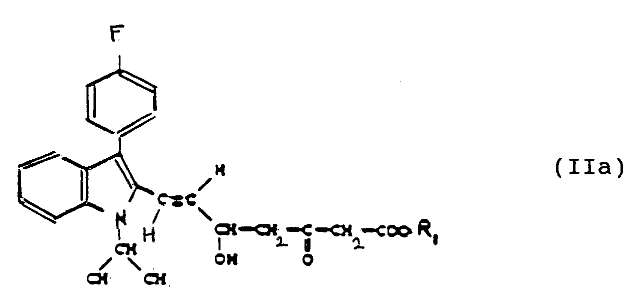 worin R₁ wie in Anspruch 1 definiert ist; hat, in razemischer oder optisch reiner Form, und wie folgt hergestellt wird:
a) daß man eine Verbindung der Formel VIIIa, wie in Anspruch 4 definiert, mit einer Verbindung der Formel IX, wie in Anspruch 4 definiert, gegebenenfalls in einem inerten wasserfreien organischen Medium umsetzt unter Bildung einer entsprechenden Verbindung der Formel Xc, wie in Anspruch 4 definiert;
b) diese Verbindung der Formel Xc mit einer Verbindung der Formel XI, wie in Anspruch 4 definiert, gegebenenfalls in einem inerten wasserfreien organischen Medium umsetzt unter Bildung einer entsprechenden Verbindung der Formel XIIc, wie in Anspruch 4 definiert;
c) diese Verbindung der Formel XIIc hydrolysiert, um eine entsprechende Verbindung der Formel VIIc, wie in Anspruch 4 definiert, in Form der freien Base oder des Säureadditionssalzes zu erhalten und, wenn die Verbindung in Form des Säureadditionssalzes vorliegt, das Säureadditionssalz mit Base neutralisiert;
d) diese Verbindung der Formel VIIc mit einer Verbindung der Formel XV, wie in Anspruch 4 definiert, oder mit einer Verbindung ausgewählt aus Oxalylchlorid oder -bromid; Phosgen oder Carbonylbromid; Phosphortrichlorid oder -tribromid; Phosphorpentachlorid oder -pentabromid; und einem Alkyl- oder Arylsulfonylchlorid oder -bromid, zum Beispiel p-Toluolsulfonylchlorid oder -bromid oder Methansulfonylchlorid oder -bromid; umsetzt unter Bildung der entsprechenden Verbindung der Formel XVI, wie in Anspruch 4 definiert, und des entsprechenden Anions, zum Beispiel ⁻PO₂(X_{b})₂;
e) diese Verbindung der Formel XVI mit 3-(4'-Fluorphenyl)-1-(1'-methylethyl)-1H-indol (die Verbindung der Formel XVII, worin R₅ und R₆ Wasserstoff sind, R₇ ein 1-Methylethylrest ist und R₈ ein p-Fluorphenylrest ist) umsetzt unter Bildung einer entsprechenden Verbindung der Formel XVIIIa: 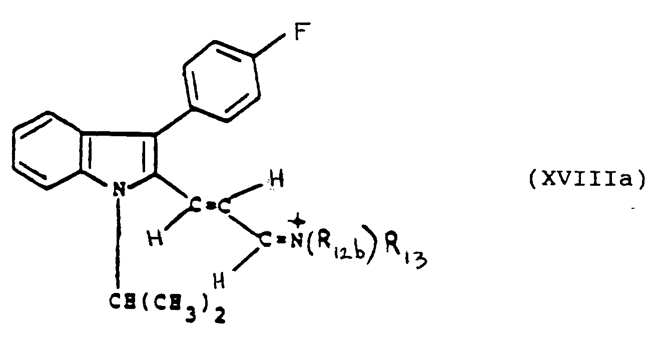 und des entsprechenden Anions, zum Beispiel ⁻PO₂(X_{b})₂; worin R_{12b}, R₁₃ und Xₐ wie in Anspruch 4 definiert sind;
f) diese Verbindung der Formel XVIIIa hydrolysiert, um (E)-3-[3'-(4''-Fluorphenyl)-1'-(1''-methy1ethyl)-1H-indol-2'-yl]-prop-2-enal (die Verbindung der Formel Va, worin R₅ und R₆ Wasserstoff sind, R₇ ein 1-Methylethylrest ist und R₈ ein p-Fluorphenylrest ist) zu erhalten; und
g) diese Verbindung der Formel Va mit dem Dianion eines Acetoessigsäureesters der Formel CH₃COCH₂COOR₁, worin R₁ wie in Anspruch 1 definiert ist, umsetzt.

10. Verfahren nach Anspruch 3, worin die Verbindung der Formel Ia in razemischer Form erhalten wird.

11. Verfahren nach Anspruch 3, worin die Verbindung der Formel Ia in Form des (3R,5S)-Enantiomers erhalten wird.

12. Verfahren nach Anspruch 3, worin die Verbindung der Formel Ia in Form des Natriumsalzes erhalten wird.

13. Verfahren nach Anspruch 4, worin R_{12b} ein Phenylrest ist und R₁₃ ein Methylrest ist.

14. Verfahren nach Anspruch 3, worin R₁ ein t-Butylrest ist.

## Revendications

1. Un procédé de préparation d'un composé de formule I 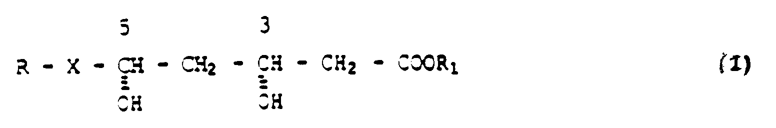 dans laquelle
X signifie -CH₂CH₂-ou-CH=CH-,
R₁ signifie un groupe ester inerte sous les conditions de la réaction, et
R signifie un radical organique ayant des groupes qui sont inertes sous des conditions de réduction,
R₁ comprenant également un groupe a!lyle et X signifiant un groupe - OCH₂-lorsque R signifie un groupe triphénylméthyle,
par réduction stéréosélective d'un composé racémique ou optiquement pur de formule II 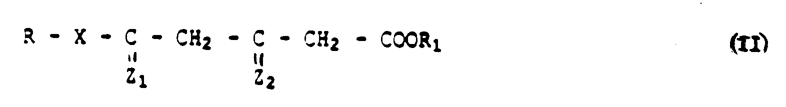 dans laquelle
R, R₁ et X sont tels que définis ci-dessus, et
l'un des symboles Z₁ et Z₂ signifie l'oxygène et l'autre signifie l'hydrogène ou un groupe hydroxy,
lequel procédé comprend
selon une première étape [étape (a)], le mélange d'un composé de formule III
R₄O-B-(R₃)₂ (III)
dans laquelle
R₄ signifie un groupe allyle ou alkyle inférieur ayant de l à 4 atomes de carbone, et
R₃ signifie un groupe alkyle primaire ou secondaire ayant de 2 à 4 atomes de carbone,
avec du borohydrure de sodium NaBH₄ dans un milieu de réaction comprenant un alcool et du tétrahydrofuranne,
selon une seconde étape [étape (b)], le traitement d'un composé de formule II par le mélange obtenu à l'étape (a) sous des conditions appropriées pour obtenir un mélange contenant un boronate cyclique de formule IV (a) 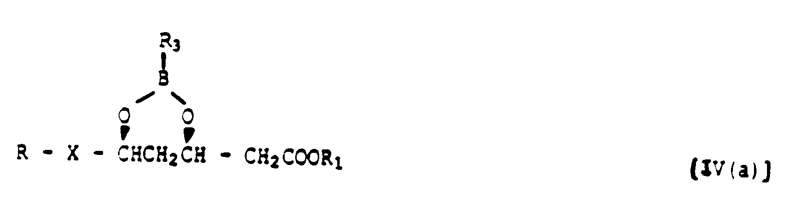 et/ou un complexe de bore de formule IV (b) 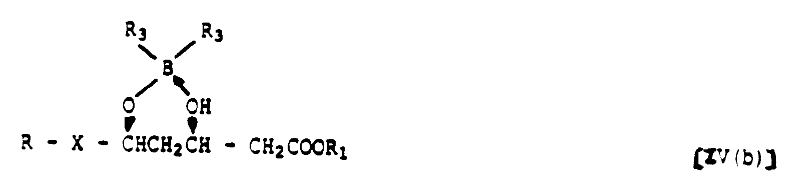 où R, R₁, R₃ et X sont tels que définis ci-dessus, et
selon une troisième étape [étape (c)], le clivage du produit obtenu à l'étape (b) pour obtenir un composé correspondant de formule I,
et, si on le désire, la transformation selon des moyens classiques du composé obtenu de formule I en acide libre, en un sel, en un autre ester ou en δ-lactone, c'est-à-dire en ester interne.

2. Un procédé selon la revendication 1 pour la préparation d'un composé de formule Iu 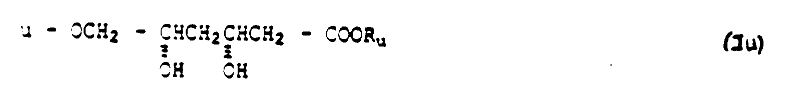 dans laquelle
u signifie un groupe triphénylméthyle (trityle), et
Rᵤ signifie un groupe allyle ou un radical formant un ester inerte sous les conditions de la réaction, de préférence un groupe allyle ou alkyle en C₁-C₃, n-butyle, iso-butyle, tert.-butyle ou benzyle, spécialement tert.-butyle,
par réduction stéréosélective d'un composé racémique ou optiquement pur de formuel IIu 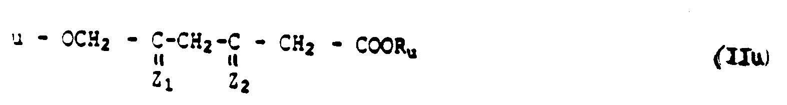 dans laquelle u, Rᵤ, Z₁ et Z₂ sont tels que définis dans la présente revendication.

3. Un procédé selon la revendication 1 pour la préparation du composé de formule Ia 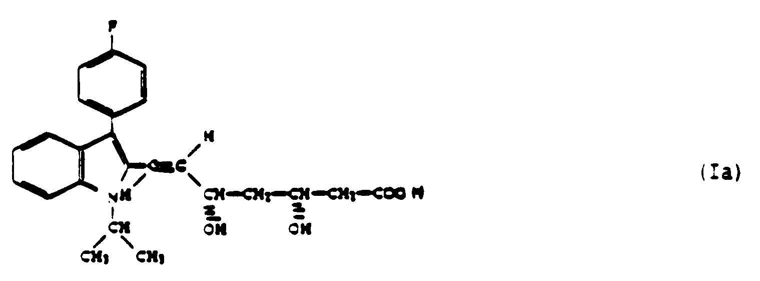 sous forme racémique ou optiquement pure; sous forme d'acide libre, de sel,
d'ester ou de δ-lactone, c'est-à-dire sous forme d'ester interne,
et comprenant, si on le désire, la transformation selon des moyens classiques du composé obtenu de formule Ia sous forme d'ester en acide libre, en sel, en un autre ester ou en δ-lactone, c'est-à-dire en ester interne.

4. Un procédé selon la revendication 1, dans lequel, pour le produit de départ de formule II, un précurseur de formule Va 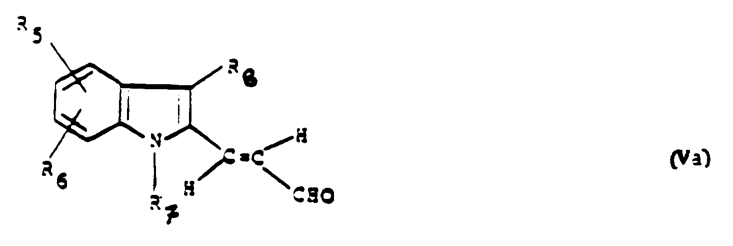 dans laquelle
R₅ signifie l'hydrogène ou un groupe alkyle en C₁-C₃, n-butyle, iso-butyle, tert.-butyle, cycloalkyle en C₃-C₆, alcoxy en C₁-C₃, n-butoxy, iso-butoxy, trifluorométhyle, fluoro, chloro, phénoxy ou benzyloxy,
R₆ signifie l'hydrogène ou un groupe alkyle en C₁-C₃, alcoxy en C₁-C₃, trifluorométhyle, fluoro, chloro, phénoxy ou benzyloxy,
un seul des symboles R₅ et R₆ pouvant signifier un groupe trifluorométhyle, un seul des symboles R₅ et R₆ pouvant signifier un groupe phénoxy et un seul des symboles R₅ et R₆ pouvant signifier un groupe benzyloxy,
un des symboles R₇ et R₈ signifie un groupe phényle trisubstitué par R₉, R₁₀ et R₁₁ et l'autre symbole signifie un groupe alkyle en C₁-C₆ primaire ou secondaire ne contenant pas d'atome de carbone asymétrique, un groupe cycloalkyle en C₃-C₆ ou un groupe phényl-(CH₂)ₘ-,
où
R₉ signifie l'hydrogène ou un groupe alkyle en C₁-C₃, n-butyle, iso-butyle, tert.-butyle, alcoxy en C₁-C₃, n-butoxy, iso-butoxy, trifluorométhyle, fluoro, chloro, phénoxy ou benzyloxy,
R₁₀ signifie l'hydrogène ou un groupe alkyle en C₁-C₃, alcoxy en C₁-C₃, trifluorométhyle, fluoro, chloro, phénoxy ou benzyloxy,
R₁₁ signifie l'hydrogène ou un groupe alkyle en C₁-C₂, alcoxy en C₁-C₂, fluoro ou chloro, et
m signifie 1, 2 ou 3,
un seul des symboles R₉ et R₁₀ pouvant signifier un groupe trifluorométhyle, un seul des symboles R₉ et R₁₀ pouvant signifier un groupe phénoxy et un seul des symboles R₉ et R₁₀ pouvant signifier un groupe benzyloxy,
est préparé comme suit:
(i) réaction d'un composé de formule VIIIa
OHC - N(2_{12b})R₁₃ (VIIIa)
dans laquelle
R_{12b} signifie un groupe phényle ou un groupe phényle portant de 1 à 3 substituants choisis chacun indépendamment parmi les groupes alkyle en C₁-C₃, alcoxy en C₁-C₃, fluoro, chloro, bromo et nitro, avec un maximum de deux groupes nitro, et
R₁₃ indépendamment a la signification indiquée ci-dessus pour R_{12b} ou signifie un groupe alkyle en C₁-C₃,
avec un composé de formule IX
Xₐ - CO-CO - Xₐ (IX)
où Xₐ signifie un groupe éliminable monovalent,
éventuellement dans un milieu organique inerte et anhydre, pour former le composé correspondant de formule Xc
Xₐ -CH=N⁺ (R_{12b})R₁₃ Xₐ⁻ (Xc)
où Xₐ, R_{12b} et R₁₃ sont tels que définis dans la présente revendication,
(ii) réaction de ce composé de formule Xc
avec un composé de formule XI
R₁₄ O - CH=CH₂ (XI)
où R₁₄ signifie un groupe monovalent qui ne désactive pas l'atome d'oxygène auquel il est fixé,
éventuellement dans un milieu organique inerte et anhydre,
pour former un composé correspondant de formule XIIc
(E) - R₁₄O - CH=CH - CH=N⁺(R_{12b})R₁₃ Xa⁻ (XIIc)
où R_{12b}, R₁₃ ,R₁₄ et Xₐ sont tels que définis dans la présente revendication, et
(iii) hydrolyse de ce composé de formule XIIc
pour obtenir un composé correspondant de formule VIIc
(E) - OHC - CH=CH- N(R_{12b})R₁₃ (VIIc)
où R_{12b} et R₁₃ sont tels que définis dans la présente revendication, sous forme de base libre ou d'un sel d'addition d'acide et, lorsqu'il est sous forme d'un sel d'addition d'acide, neutralisation du sel d'addition d'acide avec une base,
(iv) réaction de ce composé de formule VIIc
avec un composé de formule XV
PO(X_{b})₃ (XV)
où X_{b} signifie le chlore ou le brome, ou
avec un composé choisi parmi le chlorure ou bromure d'oxalyle; le phosgène ou bromure de carbonyle; le trichlorure ou tribromure de phosphore; le pentachlorure ou pentabromure de phosphore, et un chlorure ou bromure d'alkylsulfonyle ou d'arylsulfonyle tel que le chlorure ou bromure de p-toluènesulfonyle ou le chlorure ou bromure de méthanesulfonyle,
pour former le composé correspondant de formule XVI
(E)- X_{b} - CH=CH-CH=N⁺(R_{12b})R₁₃ (XVI)
et l'anion correspondant, par exemple ⁻PO₂(X_{b})₂,
où X_{b}, R_{12b} et R₁₃ sont tels que définis dans la présente revendication,
(v) réaction de ce composé de formule XVI
avec un composé de formule XVII 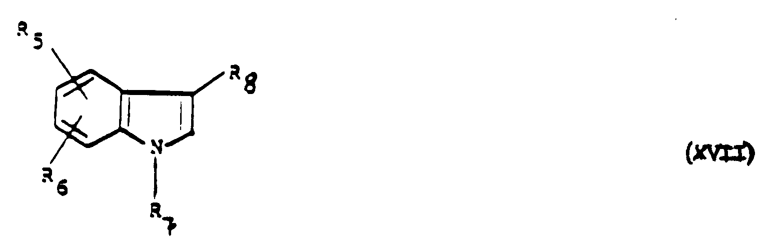 où R₅, R₆, R₇ et R₈ sont tels que définis dans la présente revendication, pour former un composé correspondant de formule XVIII 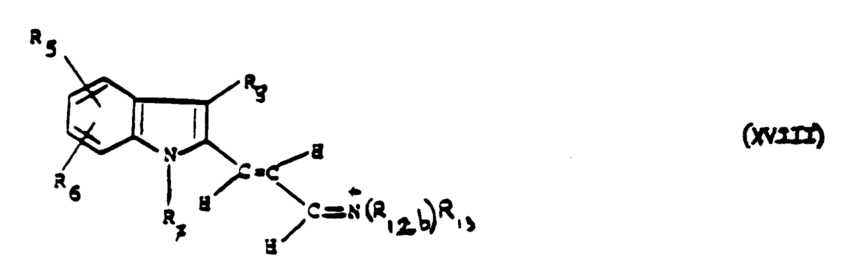 et l'anion correspondant, par exemple ⁻PO₂(X_{b})₂,
où R₅, R₆, R₇, R₈, R_{12b}, R₁₃ et X_{b} sont tels que définis dans la présente revendication, et
(vi) hydrolyse de ce composé de formule XVIII pour obtenir un composé correspondant de formule Va.

5. Un procédé selon la revendication 4, dans lequel l'étape (iii) est omise et le composé de formule XIIc est utilisé directement à la place du composé de formule VIIc pour le traitement subséquent.

6. Un procédé selon la revendication 4, dans lequel les étapes (i) et (ii) sont effectuées simultanément.

7. Un procédé selon la revendication 4, dans lequel dans les étapes (i) et/ou (ii), les produits de réaction sont utilisés purs, sans solvant.

8. Un procédé selon la revendication 4, dans lequel le composé de formule XII est sous forme d'un sel d'addition d'acide.

9. Un procédé selon la revendication 3, dans lequel le produit de départ de formule II répond à la formule IIa 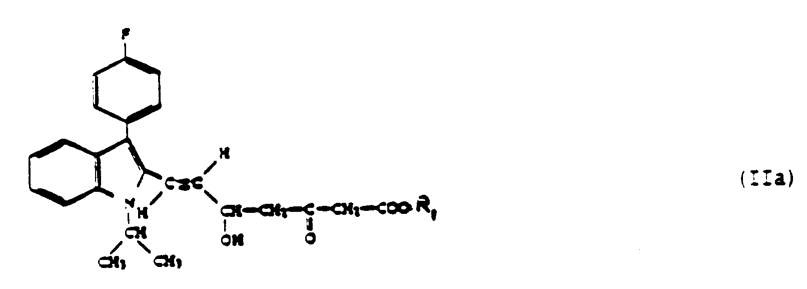 dans laquelle R₁ est tel que défini à la revendication 1; sous forme racémique ou optiquement pure,
et est préparé de la manière suivante:
a) réaction d'un composé de formule VIIIa tel que défini à la revendication 4, avec un composé de formule IX tel que défini à la revendication 4, éventuellement dans un milieu organique inerte et anhydre, pour former un composé correspondant de formule Xc tel que défini à la revendication 4,
b) réaction de ce composé de formule Xc avec un composé de formule XI tel que défini à la revendication 4, éventuellement dans un milieu organique inerte et anhydre,
pour former un composé correspondant de formule XIIc tel que défini à la revendication 4,
c) hydrolyse de ce composé de formule XIIc pour obtenir un composé correspondant de formule VIIc tel que défini à la revendication 4, sous forme de base libre ou d'un sel d'addition d'acide et, lorsqu'il est sous forme d'un sel d'addition d'acide, neutralisation du sel d'addition d'acide avec une base,
d) réaction de ce composé de formule VIIc
avec un composé de formule XV tel que défini à la revendication 4 ou avec un composé choisi parmi le chlorure ou bromure d'oxalyle; le phosgène ou bromure de carbonyle; le trichlorure ou tribromure de phosphore; le pentachlorure ou pentabromure de phosphore, et un chlorure ou bromure d'alkylsulfonyle ou d'arylsulfonyle, tel que le chlorure ou bromure de p-toluènesulfonyle ou le chlorure ou bromure de méthanesulfonyle,
pour former un composé correspondant de formule XVI tel que défini à la revendication 4 et l'anion correspondant, par exemple ⁻PO₂(X_{b})₂,
e) réaction de ce composé de formule XVI avec
le 3-(4'-fluorophényl)-1-(1 '-méthyléthyl)- 1H-indole (le composé de formule XVII où R₅ et R₆ signifient l'hydrogène, R₇ signifie un groupe 1-méthyléthyle et R₈ signifie un groupe p-fluorophényle) pour former un composé correspondant de formule XVIIIa 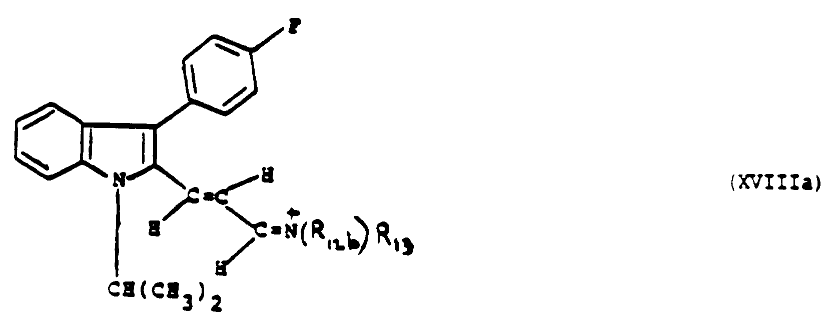 et l'anion correspondant, par exemple ⁻ PO₂ (X_{b})₂, où R_{12b}, R₁₃ et Xₐ sont tels que définis à la revendication 4,
f) hydrolyse de ce composé de formule XVIIIa pour obtenir le (E)-3-[3'-(4''-fluorophényl)- 1'-(1''-méthyléthyl)-1H-indole-2'-yl]-prop-2-énal (le composé de formule Va où R₅ et R₆ signifient l'hydrogène, R₇ signifie un groupe 1-méthyléthyle et R₈ signifie un groupe p-fluorophényle), et
g) réaction de ce composé de formule Va avec le dianion d'un ester acétoacétique de formule CH₃COCH₂COOR₁ où R₁ est tel que défini à la revendication 1.

10. Un procédé selon la revendication 3, dans lequel le composé de formule Ia est obtenu sous forme racémique.

11. Un procédé selon la revendication 3, dans lequel le composé de formule Ia est obtenu sous forme énantiomère (3R,5S).

12. Un procédé selon la revendication 3, dans lequel le composé de formule Ia est obtenu sous forme de sel de sodium.

13. Un procédé selon la revendication 4, dans lequel R_{12b} signifie un groupe phényle et R₁₃ signifie un groupe méthyle.

14. Un procédé selon la revendication 3, dans lequel R₁ signifie un groupe tert.-butyle.
